(19) Europäisches Patentamt
European Patent Office
Office européen des brevets

(11) EP 1 396 259 A2

(12) DEMANDE DE BREVET EUROPEEN

(43) Date de publication:
**10.03.2004 Bulletin 2004/11**

(51) Int Cl.7: **A61K 7/06**, A61K 7/02

(21) Numéro de dépôt: **03102701.4**

(22) Date de dépôt: **04.09.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **06.09.2002 FR 0211092**

(71) Demandeur: **L'OREAL**
**75008 Paris (FR)**

(72) Inventeurs:
- **Jager-Lezer, Nathalie**
**91000, VERRIERES-LE-BUISSON (FR)**
- **Arnaud-Roux, Mireille**
**93600, AULNAY SOUS BOIS (FR)**
- **De la Poterie, Valérie**
**77820, LE CHATELET EN BRIE (FR)**

(74) Mandataire: **Poulin, Gérard**
**BREVALEX**
**3, rue du Docteur Lancereaux**
**75008 Paris (FR)**

(54) **Composition de maquillage des fibres kératiniques**

(57) L'invention a trait à une composition de maquillage de fibres kératiniques, présentant :

- une teneur en solides définie par un extrait sec en solides supérieur à 45 % du poids total de la composition ; et
- un profil rhéologique tel que, pour une gamme de gradient de cisaillement allant $10^{-2}$ à $10^{3}$ $s^{-1}$, les rapports de la variation du gradient de cisaillement sur la variation de la contrainte de cisaillement appliquée (noté $\frac{\Delta\dot{\gamma}}{\Delta\tau}$) sont au plus égaux à 7 ; ladite composition étant apte à subir des contraintes de cisaillement sans fracturation dans toute la largeur de ladite gamme de gradient de cisaillement.

Elle permet d'accéder à un dépôt de produit sur la fibre, qui soit lisse et continu.

EP 1 396 259 A2

Printed by Jouve, 75001 PARIS (FR)

## Description

### DOMAINE TECHNIQUE

L'objet de la présente invention est une composition de maquillage pour fibres kératiniques, notamment des cils, présentant un extrait sec élevé combiné à un profil rhéologique en écoulement particulier dans le but de conduire à un dépôt de produit sur la fibre qui soit lisse et continu.

### ETAT DE LA TECHNIQUE ANTERIEURE

Les compositions de fibres kératiniques et, en particulier des cils, peuvent se présenter sous différentes formes: dispersions de solide dans liquide à phase continue hydrophile ou hydrophobe. Ces dispersions peuvent se présenter soit sous la forme d'émulsions lipophile / hydrophile ou hydrophile / lipophile ou lipophile / lipophile (les 2 phases lipophiles étant non miscibles), soit sous la forme de gel.
Ces compositions sont caractérisées par un extrait sec qui est en majeure partie une phase grasse dispersée constituée d'une ou plusieurs cires afin d'apporter de la matière sur les cils et donc un résultat maquillage plus ou moins volumateur (le maquillage des cils est plus ou moins épais).
Par ailleurs, les solides de la phase grasse peuvent également se présenter sous la forme d'agent colorant (pigment, laque, nacre) et/ou de charge pulvérulente.
Il est connu de l'art antérieur que plus l'extrait sec dans une composition augmente, plus le dépôt de matière sur le cil sera important. En conséquence, le résultat maquillage sera volumateur.
Néanmoins, l'augmentation de l'extrait sec dans une composition entraîne une augmentation de la consistance ou de la viscosité au repos (sous faible cisaillement quand le gradient de cisaillement $\dot{\gamma}$ tend vers 0). L'observation microscopique de mascaras à extrait sec important montre une mauvaise dispersion des objets solides (cires, pigment, charges).
L'augmentation de l'extrait sec est donc principalement limité par l'augmentation de consistance et ne dépasse généralement pas 44 % du poids total de la composition.
Un autre moyen pour améliorer l'effet volumateur est d'augmenter l'adhésivité du produit sur les cils en jouant sur le caractère collant de la composition pour favoriser l'accroche du mascara sur les cils et sur lui-même lors des applications successives de la brosse. Pour cela, il est possible d'utiliser des additifs dits collants (« tackifiants »), par exemple, sous la forme de cire ou de polymères filmogènes.
Il existe également des mascaras dits volumateurs qui présentent à la fois un extrait sec élevé et des additifs collants. Mais là encore, le maquillage obtenu au final est inesthétique en raison de la présence de paquets et de l'aspect granuleux du dépôt.
Il existe donc un besoin pour une composition de maquillage des fibres kératiniques alliant un extrait sec élevé avec un profil rhéologique particulier permettant ainsi un dépôt de ladite composition lisse et homogène et conférant un maquillage présentant un effet volumateur et permettant un maquillage rapide des fibres kératiniques.

### EXPOSÉ DE L'INVENTION

Le but de la présente invention est de fournir une composition de maquillage des fibres kératiniques, notamment les cils, qui réponde entre autres au besoin mentionné ci-dessus.
L'invention a ainsi pour objet des compositions de maquillage des fibres kératiniques, présentant :

- une teneur en solides définie par un extrait sec en solides supérieur à 45 % du poids total de la composition ;
- un profil rhéologique tel que, pour une gamme de gradient de cisaillement allant $10^{-2}$ à $10^{3}$ $s^{-1}$, les rapports de la variation du gradient de cisaillement sur la variation de la contrainte de cisaillement appliquée (ledit rapport étant noté

$$\frac{\Delta\dot{\gamma}}{\Delta\tau})$$

sont au plus égaux à 7, ladite composition étant apte à subir des cisaillements dans toute la largeur de ladite gamme de gradient de cisaillement. Ces compositions sont donc aptes à subir des contraintes de cisaillement sans fracturation dans toute la largeur de la gamme précitée.

La teneur en solides est mesurée conformément au protocole de mesure de la teneur en solides explicité en fin de description.

Le profil rhéologique est déterminé conformément au protocole de mesure du profil d'écoulement explicité en fin de description.

De préférence, lesdits rapports sont inférieurs à 5, de préférence inférieurs à 4,5, mieux encore inférieurs à 1,5.

Ainsi, grâce à ces caractéristiques, on accède à des compositions présentant un écoulement continu (c'est-à-dire sans fracturation) et homogène (c'est-à-dire sans formation de paquets), afin de conduire à un dépôt lisse et homogène, ceci dans une large gamme de gradient de cisaillement (de $10^{-2}$ $s^{-1}$ à $10^3$ $s^{-1}$).

La valeur maximale de la gamme de gradient de cisaillement ($10^3$ $s^{-1}$) doit être considérée en prenant en compte les incertitudes de mesure liées à l'appareil utilisé. Dans la gamme considérée, la valeur maximale de $10^3$ $s^{-1}$ doit être prise en compte avec une incertitude de mesure de $\pm$ 150 $s^{-1}$.

Avantageusement, une telle composition comporte un extrait sec en solides supérieur à 46 %, de préférence supérieur à 47 %, encore plus préférentiellement supérieur à 48 % ou mieux encore supérieur à 50 % du poids total de la composition.

Notamment, la composition a un extrait sec en solides inférieur à 85 % en poids, de préférence inférieur à 75 %, et mieux inférieur à 65 % du poids total de la composition.

Ainsi, grâce à la forte teneur globale en solides de la composition, on obtient également une composition permettant un effet épaississant et séparant satisfaisant.

Selon un premier mode de réalisation, les caractéristiques de composition précitées peuvent être obtenues par un composition comprenant au moins une phase grasse comprenant au moins un agent structurant de ladite phase grasse, qui présente avantageusement des propriétés de collant et de dureté définis.

La phase grasse totale de la composition peut représenter de 10 à 60 %, de préférence de 15 à 50%, de préférence encore de 20 à 40 % du poids total de la composition.

Un tel agent structurant de phase grasse peut être choisi parmi les cires collantes, les associations d'un composé particulier avec au moins une huile et leurs mélanges.

La présence d'une huile peut être particulièrement adaptée pour une composition de soin ou de traitement cosmétique des fibres kératiniques, en particulier des cils.

De tels agents structurants présentent l'avantage de pouvoir être incorporés à des teneurs très élevées pouvant aller jusqu'à 50% en poids de la composition sans augmenter de façon importante la consistance. En particulier, la composition peut comprendre de de 0,1 à 60 %, de préférence de 5 à 55 % en poids, de préférence encore de 10 à 40 % en poids d'agent structurant.

Il est donc possible d'incorporer ces agents structurants de phase grasse en partie ou totalité de la phase grasse sans augmenter fortement la consistance et donc d'atteindre des teneurs globales en solides beaucoup plus importantes que l'art antérieur avec un profil rhéologique conforme à la définition donnée ci-dessus, qui traduit la capacité des ces compositions à pouvoir former un dépôt lisse, homogène et continu.

Un autre avantage est que l'utilisation de ce type de composé permet également d'obtenir des compositions dont la consistance permet une application facile et homogène.

Lorsque l'un au moins des agents structurants est une cire collante, celle-ci présente avantageusement les caractéristiques suivantes :

- un collant $\geq$ 0,7 N.s, notamment allant de 0,7 à 30 N.s ; de préférence $\geq$ 1 N.s, notamment allant de 1 à 20 N.s ou mieux encore $\geq$ 2 N.s, notamment allant de 2 à 10 N.s ; et préférentiellement de 2 à 5 N.s ;
- une dureté $\leq$ 3,5 MPa, de préférence allant de 0,01 à 3,5 MPa, encore plus préférentiellement de 0,05 à 3 MPa et mieux encore de 0,1 à 2,5 MPa.

Les protocoles de mesure du collant et de la dureté sont explicités à la fin de cette description.

Selon l'invention, dans ce qui précède et ce qui suit, on entend par « cire » un composé gras lipophile, solide à température ambiante (25°C) et pression atmosphérique (760 mm de Hg, soit 105 Pa), à changement d'état solide/liquide réversible, ayant une température de fusion supérieure à 30°C et mieux supérieure à 55°C et pouvant aller jusqu'à 200°C, notamment jusqu'à 120 °C.

En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER, avec une montée en température de 5 ou 10 °C par minute.

Comme cire collante, on peut utiliser un (hydroxystéaryloxy) stéarate d' alkyle en $C_{20}$-$C_{40}$ (le groupe alkyle comprenant de 20 à 40 atomes de carbone), en particulier un 12-(12'hydroxystéaryloxy)stéarate d'alkyle en $C_{20}$-$C_{40}$, de formule (I) :

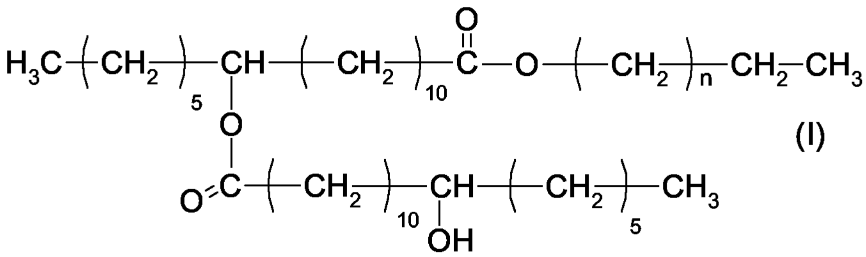

(I)

dans laquelle n est un entier allant de 18 à 38, ou un mélange de composés de formule (I).

Une telle cire est notamment vendue sous les dénominations « KESTER WAX K 82 P » et « KESTER WAX K 80 P » par la société KOSTER KEUNEN.

Lorsque l'un au moins des agents structurants est une des associations mentionnées ci-dessus (à savoir l'association d'un composé particulier avec au moins une huile), celle-ci présente avantageusement les caractéristiques suivantes:

- un collant ≥ 0,1 N.s, notamment de 0,1 à 30 N.s, de préférence ≥ 0,5 N.s, notamment de 0,5N.s à 20 N.s, mieux ≥ 0,8 N .s notamment de 0,8 à 10 N.s, et encore mieux ≥ 1, notamment entre 1 et 5 ;
- une dureté ≤ 30 MPa, notamment entre 0,01 à 30 MPa, de préférence entre 0,05 et 25 MPa, mieux entre 0,1 et 20 MPa.

Le composé particulier peut être choisi parmi les polymères semi-cristallins, les agents rhéologiques de phase grasse; et leurs mélanges.

En particulier, lorsque le composé particulier est un polymère semi-cristallin, l'association polymère semi-cristallin avec au moins une huile présente avantageusement un collant de 1 à 5 N.s et une valeur de dureté de 0,1 à 20 Mpa.

On précise que, selon l'invention, dans le cas des associations susmentionnées, on entend par « huile », un corps gras liquide à température ambiante.

On précise en outre que par « composé volatil », par exemple « huile volatile », on entend, au sens de l'invention, tout composé (ou milieu non aqueux) susceptible de s'évaporer au contact de la peau ou de la fibre kératinique en moins d'une heure, à température ambiante et pression atmosphérique. Le composé volatil est un composé cosmétique volatil, liquide à température ambiante, ayant notamment une pression de vapeur non nulle, à température ambiante et pression atmosphérique, notamment ayant une pression de vapeur allant de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par opposition, on entend par « composé non volatil », par exemple « huile non volatile », un composé restant sur la peau ou la fibre kératinique à température ambiante et pression atmosphérique, au moins plusieurs heures et ayant notamment une pression de vapeur inférieure à $10^{-3}$ mm de Hg (0,13 Pa).

L'huile peut être choisi parmi toutes les huiles physiologiquement acceptables et en particulier cosmétiquement acceptables, notamment les huiles minérales, animales, végétales, synthétiques ; en particulier les huiles hydrocarbonées et/ou siliconées et/ou fluorées volatiles ou non volatiles et leurs mélanges.

Plus précisément, par « huile hydrocarbonée », on entend une huile comportant principalement des atomes de carbone et d'hydrogène et éventuellement une ou plusieurs fonctions choisies parmi les fonctions hydroxyle, ester, éther, carboxylique. Généralement, l'huile présente une viscosité de 0,5 à 100 000 cps, de préférence de 50 à 50 000 cps et de préférence encore de 100 à 300 000 cps.

A titre d'exemple d'huiles utilisables dans l'invention, on peut citer :

- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam ;

- les esters et les éthers de synthèse notamment d'acides gras comme les huiles de formule $R_1COOR_2$ dans laquelle $R_1$ représente le reste d'un acide gras supérieur comportant de 1 à 40 atomes de carbone et $R_2$ représente une chaîne hydrocarbonée contenant de 1 à 40 atomes de carbone avec $R_1 + R_2 \geq 10$ comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle, le tridecyl trimellitate ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;
- des alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées ;
- les huiles siliconées comme les polydiméthylsiloxanes (PDMS) volatiles ou non, linéaires ou cycliques; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates,
- leurs mélanges.

De préférence, l'huile a une masse moléculaire supérieure ou égale à 250 g/mol notamment entre 250 et 10000 g/mol, de préférence supérieure ou égale à 300g/mol, notamment entre 300 et 8000 g/mol et mieux, supérieure ou égale à 400 g/mol, notamment entre 400 et 5000 g/mol. Cette huile peut être choisie parmi :

- les polybutylènes tels que l'INDOPOL H-100 (de masse molaire ou MM=965 g/mol), l'INDOPOL H-300 (MM=1340 g/mol), l'INDOPOL H-1500 (MM=2160g/mol) commercialisés ou fabriqués par la société AMOCO ;
- les polyisobutylènes hydrogénés tels que le PANALANE H-300 E commercialisé ou fabriqué par la société AMOCO (M =1340 g/mol), le VISEAL 20000 commercialisé ou fabriqué par la société SYNTEAL (MM=6000 g/mol), le REWOPAL PIB 1000 commercialisé

ou fabriqué par la société WITCO (MM=1000 g/mol) ;

- les polydécènes et les polydécènes hydrogénés tels que: le PURESYN 10 (MM=723 g/mol), le PURESYN 150 (MM=9200 g/mol) commercialisé ou fabriqués par la société MOBIL CHEMICALS,
- les esters tels que
- les esters d'acides gras linéaires ayant un nombre total de carbone allant de 30 à 70 comme le tétrapélargonate de pentaérythrityle (MM=697,05 g/mol),
- les esters hydroxylés tels que le malate de diisostéaryle (MM= 639 g/mol),
- les esters aromatiques tels que le tridecyl trimellitate (MM=757,19 g/mol),
- les esters d'alcool gras ou d'acides gras ramifiés en C24-C28 tels que ceux décrits dans la demande EP-A-0 955 039, et notamment le citrate de triisocétyle (MM= 865 g/mol), le tétraisononanoate de pentaérythrityle (MM=697,05g/mol), le triisostéarate de glycéryle (MM=891,51 g/mol), le tri décyl-2 tétradécanoate de glycéryle (MM=1143,98 g/mol), le tétraisostéarate de pentaérythrityle (MM=1202,02 g/mol), le tétraisostéarate de polyglycéryle -2 (MM=1232,04 g/mol) ou encore le tétra décyl -2 tétradécanoate de pentaérythrityle (MM=1538,66 g/mol),
- les huiles d'origine végétale telles que l'huile de sésame (820,6 g/mol), et leurs mélanges.

On précise que, selon l'invention, dans le cas des associations susmentionnées, on entend par polymère des composés comportant au moins deux motifs, de préférence au moins 3 motifs et plus spécialement au moins 10 motifs de répétition. Par "polymère semi-cristallin", on entend des polymères comportant une partie cristallisable, une chaîne pendante cristallisable ou une séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe; le polymère semi-cristallin est dans ce cas un copolymère séquencé par exemple du type dibloc, tribloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.

Le polymère semi-cristallin selon l'invention a une température de fusion supérieure ou égale à 30°C (notamment allant de 30°C à 80°C), de préférence allant de 30°C à 60°C. Cette température de fusion est une température de changement d'état du premier ordre.

Cette température de fusion peut être mesurée par toute méthode connue et en particulier à l'aide d'un calorimètre à balayage différentiel (D.S.C) .

De façon avantageuse, le ou les polymères semi-cristallins auxquelles s'applique l'invention présentent une masse moléculaire moyenne en nombre supérieure ou égale à 1 000.

De façon avantageuse, le ou les polymères semi-cristallins de la composition de l'invention ont une masse moléculaire moyenne en nombre $\overline{M}n$ allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la "chaîne pendante cristallisable" peut être chaîne comportant au moins 6 atomes de carbone.

De préférence, la ou les séquences ou chaînes cristallisables des polymères semi-cristallins représentent au moins 30 % du poids total de chaque polymère et mieux au moins 40 %. Les polymères semi-cristallins de l'invention à séquences cristallisables sont des polymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

De préférence, les polymères semi-cristallins de l'invention sont d'origine synthétique. En outre, ils ne comportent pas de squelette polysaccharidique. De façon générale, les motifs (chaînes ou séquences) cristallisables des polymères semi-cristallins selon l'invention, proviennent de monomère (s) à séquence (s) ou chaîne (s) cristallisable (s), utilisé(s) pour la fabrication des polymères semi-cristallins.

Selon l'invention, le polymère semi-cristallin peut être choisi parmi les copolymères séquencés comportant au moins une séquence cristallisable et au moins une séquence amorphe, les homopolymères et les copolymères portant au moins une chaîne latérale cristallisable par motif de répétition, leurs mélanges.

Les polymères semi-cristallins utilisables dans l'invention sont en particulier :

- les copolymères séquencés de polyoléfines à cristallisation contrôlée, notamment ceux dont les monomères sont décrits dans EP-A-0 951 897.
- les polycondensats et notamment de type polyester, aliphatique ou aromatique ou copolyester aliphatique/aromatique,
- les homo- ou co-polymères portant au moins une chaîne latérale cristallisable et les homo- ou co-polymères portant dans le squelette au moins une séquence cristallisable, comme ceux décrits dans le document US-A-5 156 911,
- les homo- ou co-polymères portant au moins une chaîne latérale cristallisable en particulier à groupement(s) fluoré (s) tels que décrits dans le document WO-A-01/19333,

et leurs mélanges. Dans ces deux derniers cas, la ou les chaînes latérales ou séquences cristallisables sont hydrophobes.

*A) Polymères semi-cristallins à chaînes latérales cristallisables*

On peut citer en particulier ceux définis dans le document US-A-5,156,911 et WO-A-01/19333. Ce sont des homopolymères ou copolymères comportant de 50 à 100 % en poids de motifs résultant de la polymérisation d'un ou plusieurs monomères porteurs de chaîne latérale hydrophobe cristallisable.

. Ces homo- ou co-polymères sont de toute nature du moment qu'ils présentent les conditions indiquées précédemment.

Ils peuvent résulter:

- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
- de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

D'une façon générale, ces polymères sont choisis notamment parmi les homopolymères et copolymères résultant de la polymérisation d'au moins un monomère à chaîne (s) cristallisable (s) qui peut être représenté par la formule X :

```
— M —
  |
  S
  |
  C
```

avec M représentant un atome du squelette polymérique, S représentant un espaceur, C représentant un groupe cristallisable.

Les chaînes « -S-C » cristallisables peuvent être aliphatiques ou aromatiques, éventuellement fluorées ou perfluorées. « S » représente notamment un groupe $(CH_2)_n$ ou $(CH_2CH_2O)_n$ ou $(CH_2O)$, linéaire ou ramifié ou cyclique, avec n entier allant de 0 à 22. De préférence « S » est un groupe linéaire. De préférence, « S » et « C » sont différents.

Lorsque les chaînes « -S-C » cristallisables sont des chaînes aliphatiques hydrocarbonées, elles comportent des chaînes alkyle hydrocarbonées à au moins 11 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyles en $C_{14}$-$C_{24}$. Lorsqu'il s'agit de chaînes alkyle fluorées ou perfluorées, elles comportent au moins 6 atomes de carbone fluorés et notamment au moins 11 atomes de carbone dont au moins 6 atomes de carbone sont fluorés.

Comme exemple de polymères ou copolymères semi-cristallins à chaîne (s) cristallisable (s) , on peut citer ceux résultant de la polymérisation d'un ou plusieurs monomères suivants: les (méth)acrylates d'alkyle saturés avec le groupe alkyle en $C_{14}$-$C_{24}$, les (méth)acrylates de perfluoroalkyle avec un groupe alkyle perfluoro en $C_{11}$-$C_{15,}$ les N-alkyl (méth)acrylamides avec le groupe alkyle en $C_{14}$ à $C_{24}$ avec ou sans atome de fluor, les esters vinyliques à chaînes alkyle ou perfluoro (alkyle) avec le groupe alkyle en $C_{14}$ à $C_{24}$ (avec au moins 6 atomes de fluor pour une chaîne perfluoro alkyle), les éthers vinyliques à chaînes alkyle ou perfluoro (alkyle) avec le groupe alkyle en $C_{14}$ à $C_{24}$ et au moins 6 atomes de fluor pour une chaîne perfluoro alkyle, les alphaoléfines en $C_{14}$ à $C_{24}$ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères objets de l'invention sont des copolymères, ils contiennent, en plus, de 0 à 50 % de groupes Y ou Z résultant de la copolymérisation :

$\alpha$ ) de Y qui est un monomère polaire ou non polaire ou un mélange des deux :

- Lorsque Y est un monomère polaire, c'est soit un monomère porteur de groupes polyoxyalkylénés (notamment oxyéthyléné et/ou oxypropyléné), un (méth)acrylate d'hydroxyalkyle comme l'acrylate d'hydroxyéthyle, le (méth)acrylamide, un N-alkyl(méth)acrylamide, un N,N-dialkyl(méth)acrylamide comme par exemple le N,N-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.

- Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou styrène substitué par un groupe alkyle en $C_1$ à $C_{10,}$ comme l'$\alpha$-méthylstyrène, un macromonomère du type polyorganosiloxane à insaturation vinylique.

Par "alkyle», on entend au sens au sens de l'invention un groupement saturé notamment en $C_8$ à $C_{24}$, sauf mention exprès, et mieux en $C_{14}$ à $C_{24}$.

$\beta$) de Z qui est un monomère polaire ou un mélange de monomères polaires. Dans ce cas, Z a la même définition que le "Y polaire" défini ci-dessus.

De préférence, les polymères semi-cristallins à chaîne latérale cristallisable sont des homopolymères d'alkyl (méth)acrylate ou d'alkyl(méth)acrylamide avec un groupe alkyle tel que défini ci-dessus, et notamment en $C_{14}$-$C_{24}$,

des copolymères de ces monomères avec un monomère hydrophile de préférence de nature différente de l'acide (méth)acrylique comme la N-vinylpyrrolidone ou l'hydroxyéthyl (méth)acrylate et leurs mélanges.

*B) Les polymères portant dans le squelette au moins une séquence cristallisable*

Ces polymères sont notamment des copolymères séquencés constitués d'au moins 2 séquences de nature chimique différente dont l'une est cristallisable.

- On peut utiliser les polymères séquencés définis dans le brevet US-A-5,156,911 ;
- Les copolymères séquencés d'oléfine ou de cyclooléfine à chaîne cristallisable comme ceux issus de la polymérisation séquencée de :

  . cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges,
  . avec l'éthylène, le propylène, le 1-butène, le 3-méthyl-1-butène, le 1-hexène, le 4-méthyl-1-pentène, le 1-octène, le 1-décène, le 1-éicosène ou leurs mélanges,
  . et en particulier les copoly(éthylène/norbornène) blocs et les terpolymères (éthylène/propylène/éthylidène-norbornène) blocs. On peut aussi utiliser ceux résultants de la copolymérisation séquencée d'au moins 2 α-oléfines en $C_2$-$C_{16}$ et mieux en $C_2$-$C_{12}$ et encore mieux en $C_4$-$C_{12}$ tels que ceux cités précédemment et en particulier les bipolymères séquencés d'éthylène et d'1-octène

- Les copolymères peuvent être des copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes:

  . Séquence cristallisable par nature : a) polyester comme les poly(alkylène téréphtalate), b) polyoléfine comme les polyéthylènes ou polypropylènes.
  . Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe distinctes, on peut citer:

α) les copolymères séquencés poly (μ-caprolactone)-b-poly(butadiène), utilisés de préférence hydrogénés, tels que ceux décrits dans l'article "Melting behavior of poly(μ-caprolactone)-block-polybutadiène copolymers" de S. Nojima, Macromolécules, 32, 3727-3734 (1999) .
β) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34, 117-123 (1995).
γ) les copolymères séquencés poly(éthylène)-b-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993) et " Polymer agregates with crystalline cores: the system poly(ethylene)-poly(ethylene-propylene)" de P. Richter et al., Macromolécules, 30, 1053-1068 (1997).
δ) les copolymères séquencés poly(éthylène)-b-poly(éthyléthylène) cités dans l'article général "Cristallization in block copolymers" de I.W. Hamley, Advances in Polymer Science, vol 148, 113-137 (1999).

Les polymères semi-cristallins de la composition de l'invention peuvent être ou non réticulés en partie du moment que le taux de réticulation ne gène pas leur dissolution ou dispersion dans la phase grasse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère; soit à une séparation de phase entre les séquences cristallisables et les séquences

amorphes, portées par le polymère.

De préférence, les polymères semi-cristallins de la composition selon l'invention sont non réticulés.

Selon un mode particulier de réalisation de l'invention, le polymère est choisi parmi les copolymères résultant de la polymérisation d'au moins un monomère à chaîne cristallisable choisi parmi les (méth)acrylates d'alkyle saturés en $C_{14}$ à $C_{24}$, les (méth)acrylates de perfluoroalkyle en $C_{11}$ à $C_{15}$, les N alkyl (méth) acrylamides en $C_{14}$ à $C_{24}$ avec ou sans atome de fluor, les esters vinyliques à chaînes alkyle ou perfluoroalkyle en $C_{14}$ à $C_{24}$, les éthers vinyliques à chaînes alkyle ou perfluoroalkyle en $C_{14}$ à $C_{24}$, les alphaoléfines en $C_{14}$ à $C_{24}$, les para-alkyl styrènes avec un groupe alkyle comportant de 12 à 24 atomes de carbone, avec au moins un ester ou amide d'acide monocarboxylique en $C_1$ à $C_{10}$ éventuellement fluoré, qui peut être représenté par la formule suivante:

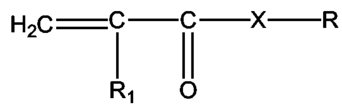

dans laquelle $R_1$ est H ou $CH_3$, R représente un groupe alkyle en $C_1$-$C_{10}$ éventuellement fluoré et X représente O, NH ou $NR_2$, où $R_2$ représente un groupe alkyle en $C_1$-$C_{10}$ éventuellement fluoré.

Selon un mode plus particulier de réalisation de l'invention, le polymère est issu d'un monomère à chaîne cristallisable choisi parmi les (méth)acrylates d'alkyle saturés en $C_{14}$ à $C_{22}$.

A titre d'exemple particulier de polymère semi-cristallin structurant utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C). Ils sont porteurs de chaînes latérales cristallisables et présentent la formule X précédente.

Les polymères semi-cristallins peuvent être notamment : ceux décrits dans les exemples 3, 4, 5, 7, 9, 13 du brevet US-A-5,156,911 à groupement -COOH, résultant de la copolymérisation d'acide acrylique et d' alkyl (méth) acrylate en $C_5$ à $C_{16}$ et plus particulièrement de la copolymérisation :

- d'acide acrylique, d'hexadécylacrylate et d'isodécylacrylate dans un rapport pondéral 1/16/3,
- d'acide acrylique et de pentadécylacrylate dans un rapport pondéral 1/19,
- d'acide acrylique, d'hexadécylacrylate, éthylacrylate dans un rapport pondéral 2,5/76,5/20,
- d'acide acrylique, d'hexadécylacrylate et de méthylacrylate dans un rapport pondéral 5/85/10,
- d'acide acrylique et de octadécylméthacrylate dans un rapport pondéral 2,5/97,5 ,
- d'hexadécylacrylate, de monométhyl éther de méthacrylate polyéthylèneglycol à 8 motifs d'éthylèneglycol, et d'acide acrylique dans un rapport pondéral 8,5/1/0,5.

On peut aussi utiliser la structure « O » de National Starch tel que celui décrit dans le document US-A-5,736,125 de point de fusion 44°C ainsi que les polymères semi-cristallins à chaînes pendantes cristalisables comportant des groupements fluorés tels que décrits dans les exemples 1, 4, 6, 7 et 8 du document WO-A-01/19333.

On peut encore utiliser les polymères semi-cristallins obtenus par copolymérisation d'acrylate de stéaryle et d'acide acrylique ou de NVP tels que décrits dans le document US-A-5 519 063 ou EP-A-550745 de température de fusion respectivement de 40°C et 38°C.

On peut aussi utiliser les polymères semi-cristallins obtenus par copolymérisation de l'acrylate de béhényle et de l'acide acrylique ou de NVP tels que décrits dans les documents US-A-5,519,063 et EP-A-550745, de température de fusion respectivement de 60°C et 58°C.

De préférence, les polymères semi-cristallins ne comportent pas de groupement carboxylique.

Les autres associations susmentionnées peuvent comprendre l'association d'un agent rhéologique avec au moins une huile.

Cet agent rhéologique est capable d'épaissir et/ou gélifier la phase huile. Il peut être présent en une quantité efficace pour augmenter la viscosité de cette phase, notamment jusqu'à l'obtention d'un gel solide, à savoir un produit ne s'écoulant pas sous son propre poids.

Cet agent rhéologique est avantageusement choisi parmi les gélifiants lipophiles, les organogélateurs et leurs mélanges.

Le gélifiant lipophile peut être organique

ou minéral, polymérique ou moléculaire.

Comme gélifiant lipophile minéral, on peut citer les argiles éventuellement modifiées comme les hectorites modifiées par un chlorure d'ammonium d'acide gras en $C_{10}$ à $C_{22}$, comme l'hectorite modifiée par du chlorure de di-stéaryl di-méthyl ammonium.

On peut églement citer la silice pyrogénée éventuellement traitée hydrophobe en surface dont la taille des particules est inférieure à 1 μm. Il est en effet possible de modifier chimiquement la surface de la silice, par réaction chimique générant une diminution du nombre de groupes silanol présents à la surface de la silice. On peut notamment substituer des groupes silanol par des groupements hydrophobes: on obtient alors une silice hydrophobe. Les groupements hydrophobes peuvent être:

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références "AEROSIL R972®", "AEROSIL R974® par la société Degussa, "CAB-O-SIL TS-610® "CAB-O-SIL TS-720® par la société Cabot.

La silice pyrogénée hydrophobe présente de préférence une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les noms KSG6, KSG16, KSG18 de Shin-Etsu, Trefil E-505C ou Trefil E-506C de Dow-Corning, Gransil SR-CYC, SR DMF10, SR-DC556, SR 5CYC gel, SR DMF 10 gel, SR DC 556 gel de Grant Industries, SF 1204 et JK 113 de General Electric ; l'éthylcellulose comme celles vendues sous le nom d'Ethocel par Dow Chemical ; les polyamides tels que les copolymères d'un diacide en $C_{36}$ condensé sur l'éthylène diamine de masse moléculaire moyenne en poids d'environ 6000 tels que les composés commercialisés par la société Arizona Chemical sous les noms Uniclear 80 et Uniclear 100, les gommes notamment siliconées comme les PDMS ayant une viscosité > 100 000 centistokes, les galactommananes comportant de un à six et mieux de deux à quatre groupes hydroxyle par ose, substitués par une chaîne alkyle saturée ou non, comme la gomme de guar alkylée par des chaînes alkyle en $C_1$ à $C_6$ et mieux en $C_1$ à $C_3$ et leurs mélanges.

Comme gélifiant lipophile préféré, on utilise des gélifiants organique moléculaires non polymériques, également appelés organogélateurs, qui sont des composés dont les molécules sont capables d'établir entre elles des interactions physiques conduisant à une auto-agrégation des molécules avec formation d'un réseau supra-moléculaire 3D qui est responsable de la gélification de la phase grasse liquide.

Par "phase grasse liquide", on entend, au sens de l'invention, une phase grasse liquide à température ambiante (25° C) et pression atmosphérique (760 mm de Hg, soit 105 Pa), composée d'un ou plusieurs corps gras liquides température ambiante, appelés aussi huiles, généralement compatibles entre eux.

Le réseau supra-moléculaire peut résulter de la formation d'un réseau de fibrilles (dues aux empilements ou agrégations de molécules d'organogélateur), immobilisant les molécules de la phase grasse liquide.

L'aptitude à former ce réseau de fibrilles, et donc à gélifier, dépend de la nature (ou classe chimique) de l'organogélateur, de la nature des substituants portés par ses molécules pour une classe chimique donnée et de la nature de la phase grasse liquide.

Les interactions physiques sont diverses mais excluent la co-cristallisation. Ces interactions physiques sont en particulier des interactions du type interactions hydrogènes auto-complémentaires, interactions π entre cycles insaturés, interactions dipolaires, liaisons de coordination avec des dérivés organométalliques et leurs associations. En général, chaque molécule d'un organogélateur peut établir plusieurs types d'interactions physiques avec une molécule voisine. Aussi, avantageusement, les molécules des organogélateurs selon l'invention comportent au moins un groupement capable d'établir des liaisons hydrogènes et mieux au moins deux groupements capables d'établir des liaisons hydrogène, au moins un cycle aromatique et mieux aux moins deux cycles aromatiques, au moins une ou plusieurs liaisons à insaturation éthylénique et/ou au moins un ou plusieurs carbones asymétriques. De préférence, les groupements capables de faire des liaisons hydrogènes sont choisis parmi les groupements hydroxyle, carbonyle, amine, acide carboxylique, amide, urée, benzyle et leurs associations.

Le ou les organogélateurs selon l'invention sont solubles dans la phase grasse liquide après chauffage jusqu'à obtention d'une phase liquide homogène transparente. Ils peuvent être solides ou liquides à température ambiante et pression atmosphérique.

Le ou les organogélateurs moléculaires utilisables dans la composition selon l'invention sont notamment ceux décrits dans le document "Specialist Surfactants", édité par D. Robb de 1997, p.209-263, chapitre 8 de P. Terech, les demandes européennes EP-A-1068854 et EP-A-1086945 ou encore dans la demande WO-A-02/47031.

On peut notamment citer parmi ces organogélateurs, les amides d'acides carboxyliques en particulier les

acides tri-carboxyliques comme les cyclohexanetricarboxamides (voir la demande de brevet européen EP-A-1068854), les diamides ayant des chaînes hydrocarbonées contenant chacune de 1 à 22 atomes de carbone, par exemple de 6 à 18 atomes de carbone, lesdites chaînes étant non substituées ou substituées avec au moins un substituant choisi parmi les groupes ester, urée et fluoro (voir la demande EP-A-1086945) et notamment les diamides résultant de la réaction du diaminocyclohexane, en particulier du diaminocyclohexane sous forme trans, et d'un chlorure d'acide comme par exemple le N,N'-bis (dodécanoyl)-1,2-diaminocyclohexane, les amides de N-acylamino acides comme les diamides résultant de l'action d'un N-acylamino acide avec des amines comportant de 1 à 22 atomes de carbone, comme par exemple ceux décrits dans le document WO-93/23008 et notamment les amides de l'acide N-acylglutamique où le groupe acyle représente une chaîne alkyle en $C_8$ à $C_{22}$ tels que le dibutylamide de l'acide N-Lauroyl-L-glutamique, fabriqué ou commercialisé par la société Ajinomoto sous la dénomination GP-1 et leurs mélanges.

Les compositions peuvent contenir de 1 à 60% d'agent structurant compris dans la phase grasse. De préférence, la composition contient de 5 à 55% en poids, mieux de 10 à 40% d'agent structurant, ce qui permet d'atteindre des teneurs globales en solides dans la composition supérieure à 45%, de préférence supérieure à 46%, mieux supérieure à 47%, encore mieux supérieure à 48%, voire supérieure à 50%.

Un exemple d'un agent structurant de type « cire collante » correspondant à l'invention est le suivant: 12-(12'-hydroxystéaryloxy)stéarate d'alkyle en C20-C40 Nom commercial : Kester wax K82P et Kester wax K 80P de chez Koster Keunen

Collant = 3,38 N.s

Dureté = 0,96 MPa

Un exemple d'un agent structurant de type 2 correspondant à l'invention constitué d'un polymère semi-cristallin associé à une huile est le suivant : Phase grasse = mélange polybutène(l) / cop.acrylate de stéaryle N-vinyl pyrrolidonne(2) (40/60)de point de fusion de 56°C.

(1) : Indopol H 100 de la société AMOCHO
(2) : Polymère basique de point de fusion de 56°C préparé selon le mode opératoire suivant

Dans un réacteur d'11 muni d'une agitation centrale avec ancre, d'un réfrigérant et d'un thermomètre, on introduit 120 g de cyclohexane que l'on chauffe de la température ambiante à 80°C en 45 min. A 80°C, on introduit en 2h le mélange $C_1$ suivant:

40 g de cyclohexane + 4 g de Triganox 141 [2,5 bis (2-éthyl hexanoyl peroxy) - 2,5 - diméthyl hexane].

30 min après le début de la coulée du mélange $C_1$, on introduit en 1h30 le mélange $C_2$ constitué de :

190 g d'acrylate de stéaryle + 10 g de N-vinyl pyrrolidone + 400 g de cyclohexane.

A la fin des deux coulées, on laisse agir 3h supplémentaires à 80°C puis on distille à pression atmosphérique la totalité du cyclohexane présent dans le milieu réactionnel.

On obtient alors le polymère à 100 % en poids en matière active.

Sa masse moléculaire moyenne en poids $M_w$ est de 38 000 exprimée en équivalent polystyrène et sa température de fusion pF est de 56°C, mesurée par D.S.C.

Collant = 2,63 N.s
Dureté = 5,84 MPa

Selon un deuxième mode de réalisation de l'invention, la composition présentant les caractéristiques précitées, peut être obtenue en incorporant dans une phase aqueuse au moins un agent structurant de phase aqueuse permettant d'obtenir le profil rhéologique défini précédemment.

L'agent structurant de phase aqueuse peut être choisi parmi les tensioactifs non ioniques et anioniques conduisant à la formation de phases lamellaires, les polymères amphiphiles conduisant à la formation de phases lamellaires, les polymères associatifs.

Selon l'invention, on précise que par 'phases lamellaires', on entend des composés amphiphiles possédant la propriété de former, en présence d'eau, des phases mésomorphes dont l'état d'organisation est intermédiaire entre

l'état cristallin et l'état liquide. Parmi les amphiphiles qui donnent naissance à des phases mésomorphes, certains peuvent gonfler en solution aqueuse pour former soit des sphérules dispersées en solution, soit des feuillets: ces organisations sont constituées par des couches bi-moléculaires

Les phases lamellaires se présentent classiquement sous une forme de feuillets, encore appelés bicouches, ou sous une forme enroulée, encore appelée vésicules, sphérules ou phases « oignons ».

Toutes phases lamellaires se caractérisent en microscopie sous lumière polarisée, par une structure biréfringente soit sous forme de stries huileuses ou de croix de malte, encore appelée croix de polarisation.

Exemple d'amphiphiles pouvant conduire à des phases lamellaires :

Les molécules amphiphiles peuvent être de nature ionique, de préférence anionique, non ionique, amphotère ou zwitterionique et leur combinaison.

→ Sont capables de former des phases lamellaires un amphiphile plutôt lipophile (HLB = 1-7) ou un amphiphile plutôt hydrophile (HLB = 8-20) ou bien un mélange des 2.

• Les tensioactifs hydrocarbonés

A titre d'exemples de tensioactifs hydrocarbonés, on peut citer les lipides amphiphiles utilisés pour l'obtention des vésicules sont des lipides ayant pour formule générale:

$$X - Y$$

formule dans laquelle X représente un groupe hydrophile et Y représente un groupe lipophile. Les lipides amphiphiles peuvent être des lipides ioniques, pour lesquels le groupe X est ionique, ou des lipides non-ioniques pour lesquelles le groupe X est non-ionique.

Les lipides amphiphiles ioniques sont, de préférence, choisis dans le groupe formé par les phospholipides naturels, modifiés par voie chimique ou enzymatique ou de synthèse, les composés anioniques et les gangliosides.

Parmi les phospholipides naturels, on peut citer la lécithine d'oeuf ou de soja, et la sphingomyéline; parmi les phospholipides de synthèse on peut citer la dipalmitoyl-phosphatidylcholine et parmi les phospholipides-modifiés, on peut citer la lécithine hydrogénée.

Parmi les composés anioniques, on peut citer ceux qui sont représentés par la formule :

$$R_1-CHOH-\underset{\displaystyle R_2CONH}{\underset{|}{CH}}-COOM$$

Formule dans laquelle :

- $R_1$ représente un radical alkyle ou alcényle en $C_7$ - $C_{21}$ ;
- $R_2$ représente un radical hydrocarboné, saturé ou insaturé, en $C_7$ - $C_{31}$ ; et
- M représente H, Na, K, NH4 ou un ion ammonium substitué dérivé d'une amine.

Les lipides amphiphiles non-ioniques sont, de préférence, choisis dans le groupe formé par:

(1) les dérivés de polyglycérol, linéaires ou ramifiés, de formule:

$$R_3O-\left[C_3H_5(OH)O\right]_n-H$$

dans laquelle:

$-C_3H_5(OH)O-$ est représenté par les structures suivantes prises en mélange ou séparément:

- $CH_2CHOHCH_2O-$;

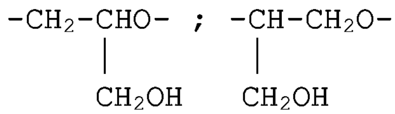

n est une valeur statistique moyenne comprise entre 2 et 6 :

$R_3$ représente :

(a) une chaîne aliphatique, linéaire ou ramifiée, saturée ou insaturée, contenant de 12 à 30 atomes de carbone ; ou les radicaux hydrocarbonés des alcools de lanoline ;
(b) un reste $R'_3CO$, où $R'_3$ est un radical aliphatique, linéaire ou ramifié, en $C_{11}$-$C_{17}$ ;
(c) un reste

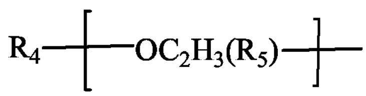

où

- . $R_4$ peut prendre la signification (a) ou (b) donnée pour $R_3$ ;
- . $-OC_2H_3$ $(R_5)$- est représenté par les structures suivantes, prises en mélange ou séparément :

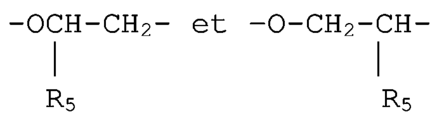

où $R_5$ prend la signification (a) donnée pour $R_3$ ;

(2) les éthers de polyglycérol, linéaires ou ramifiés, comportant deux chaînes grasses ;
(3) les alcools gras polyoxyéthylénés et les stérols et phytostérols polyoxyéthylénés ;
(4) les éthers de polyols ;
(5) les esters de polyols, oxyéthylénés ou non ;
(6) les glycolipides d'origine naturelle ou synthétique ;
(7) les hydroxyamides représentés par la formule :

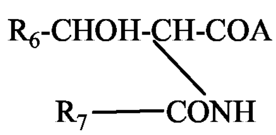

dans laquelle:

- $R_6$ désigne un radical alkyle ou alcényle en $C_7$-$C_{21}$ ;
- $R_7$ désigne un radical hydrocarboné, saturé ou insaturé, en $C_7$-$C_{31}$ ;
- COA désigne un groupement choisi parmi les deux groupements suivants:

. un reste

CON-B
|
$R_8$

où :

B est un radical dérivé d'amines primaires ou secondaires, mono- ou polydroxylées ; et
$R_8$ désigne un atome d'hydrogène ou un radical méthyle, éthyle ou hydroxyéthyle ; et

. un reste -COOZ, ou Z représente le reste d'un polyol en $C_3$-$C_7$.

Dans les dérivés de polyglycérol, le radical R aliphatique linéaire saturé est, de préférence, un radical lauryle, myristyle, cétyle, stéaryle, arachidyle, bénéhyle, lignocéryle ou un mélange de ces radicaux ; et le radical $R_3$ aliphatique non saturé est avantageusement un radical palmitoléyle, oléyle, linoléyle, arachidonyle.

Les composés définis au point (3) ci-dessus sont avantageusement des alcools en $C_{12}$ à $C_{22}$ portant 2 à 20 unités oxyde d'éthylène (O.E). Le stérol est, avantageusement le cholestérol ; il peut être substitué par 2 à 20 unités d'O.E. De même le phytostérol peut être substitué par 2 à 20 moles d'O.E.

Les éthers de polyols définis au point (4) ci-dessus sont, de préférence, des alkyl éthers de polyol en $C_2$ à $C_7$.

Les esters de polyols définis au point (5) ci-dessus non-ioniques utilisables comme lipides amphiphiles non-ioniques sont avantageusement les cérébrosides.

On peut, selon l'invention, incorporer dans la phase lipidique des additifs, qui permettent de diminuer la perméabilité des vésicules, et/ou des lipides chargés destinés à améliorer la stabilité des vésicules, en prévenant leur floculation et leur fusion, et à permettre l'augmentation du taux d'encapsulation.

On peut notamment associer aux lipides amphiphiles constitutifs des vésicules au moins un additif choisi dans le groupe formé par :

- les stérols et leurs dérivés par exemple oxyéthylénés, plus particulièrement le cholestérol, le sulfate de cholestérol acide et ses sels alcalins et le phosphate de cholestérol acide et ses sels alcalins ;
- les alcools et diols à longue chaîne ;
- les amines à longue chaîne et leurs dérivés ammonium quaternaire ;
- les dihydroxyalylamines ;
- les amines grasses polyoxyéthylénées ;
- les esters d'aminoalcools à longue chaîne et leurs sels et dérivés ammonium quaternaire ;
- les esters phosphoriques d'alcools gras, par exemple le dicéthylphosphate et le dimyristylphosphate sous forme acides ou de sels alcalins.

Les dispersions de vésicules peuvent contenir un ou plusieurs composé (s) actif (s) ayant une activité cosmétique et/ou dermopharmaceutique, qui, selon leurs caractéristiques de solubilité, peuvent avoir différentes localisations. Si les actifs sont liposolubles, on les introduits dans la phase lipidique constituant le(s) feuillet(s) des vésicules. Si les actifs sont hydrosolubles, on les introduits dans la phase aqueuse encapsulée des vésicules, si les actifs sont amphiphiles, ils se répartissent entre la phase lipidique et la phase aqueuse encapsulée avec un coefficient de partage qui varie selon la nature de l'actif amphiphile et les compositions respectives de la phase lipidique et de la phase aqueuse encapsulée.

Des actifs hydrosolubles sont, par exemple, la glycérine, le sorbitol, l'érythrulose et les antibiotiques ; des actifs liposolubles sont par exemple l'acide rétinoïque, les lipoprotides et les stéroïdes.

On peut citer également en tant que tensioactifs hydrocarbonés les produits ayant une formule statistique, dans leur partie hydrophile, par exemple un ester de polyglycérol de formule

$$R\,CO\left[-OCH_2\text{-}CHOH\text{-}CH_2-\right]_n OH$$

où n est une valeur statistique et qui peut contenir des proportions diverses d'esters pour lesquels n-1, n-2, n-3, n-4, etc... ; c'est aussi le cas des esters comportant plusieurs chaînes alkyle dans leur partie lipophile, tels que les cocoates,

qui contiennent des chaînes alkyle en $C_5$ à $C_{17}$ ou les isostéarates, où les chaînes alkyle en $C_{17}$ sont un mélange complexe de formes isomères, c'est également le cas des produits constitués par des mélanges de mono-, di-, tri-ou polyesters d'un même polyol. Il faut noter qu'un produit, qui ne contiendrait qu'un seul ester susceptible de former des vésicules et des impuretés d'un autre type ne pourrait pas être utilisé selon l'invention.

Des esters commerciaux utilisables seuls selon l'invention, car ils sont en réalité des mélanges d'esters, sont par exemple les suivants :

- les esters partiels de sorbitane (ou abhydride de sorbitol) et l'acide gras, vendus sous les dénominations commerciales « SPAN 20, 40, 60 et 80 » par la société « ICI » ;
- l'isostéarate de sorbitane, vendu sous la dénomination commerciale « SI 10 R NIKKOL» par la société « NIKKO » ;
- le stéarate de sorbitane portant 4 unités oxyde d'éthylène vendu sous la dénomination « TWEEN 61 » par la société « ICI » ;
- le stéarate de polyéthylène glycol à 8 unités oxyde d'éthylène, vendu sous la dénomination « MYR J 45 » par la société « ICI » ;
- le monostéarate du polyéthylène glycol de formule :

$$OHCH_2(CH_20CH_2)_nCH_2OH$$

  formule dans laquelle n est égal à 4, vendu sous la dénomination « MYS 4 » par la société « NIKKO » ;
- le stéarate de polyéthylène glycol de poids moléculaire 400, qualité chimique ou qualité produite par biotechnologie, vendu par la société « UNICHEMA » ;
- le stéarate de diglycéryle portant 4 unités d'oxyde d'éthylène, vendu sous la dénomination « HOSTACERINE DGS » par la société « HOECHST » ;
- le stéarate de tétraglycérol, vendu sous la dénomination « TETRAGLYN 1S » par la société « NIKKO » ;
- l'isostéarate de diglycéryle, vendu par la société « SOLVAY » ;
- le distéarate de diglycéryle, vendu sous la dénomination « EMAILEX DSG 2 » par la société « NIHON » ;
- les mono-, di- et tripalmitostéarate de sucrose, vendus sous les dénominations « F50, F70, F110 et F160 CRODESTA » par la société « CRODA » ;
- le mélange de mono- et dipalmitostéarate de sucrose, vendu sous la dénomination « GRILLOTEN PSE 141 G » par la société « GRILLO » ;
- le mélange de stéarate de sucrose et de cocoate de sucrose, vendu sous la dénomination « ARLATONE 2121 » par la société « ICI » ;
- le distéarate de méthylglucose portant 20 unités oxyde d'éthylène, vendu sous la dénomination « GLUCAM E 20 DISTEARATE » par la société « AMERCHOL ».

Des mélanges entre eux de ces différents produits, qui sont déjà des mélanges, ou des mélanges de ces produits avec des produits purs peuvent, bien entendu, être utilisés.

Le (s) lipides) amphiphile (s) ionique (s) associé(s) aux lipides amphiphiles non-ioniques selon l'invention, est(sont), de préférence, pris dans le groupe formé par :

1) Les lipides anioniques neutralisés, ces lipides anioniques étant, de préférence, choisis parmi :

- les sels alcalins du dicétylphosphate, et du dimyristylphosphate en particulier les sels de Na et K ;
- les sels alcalins du cholestérol sulfate, en particulier le sel de Na ;
- les sels alcalins du cholestérol-phosphate, en particulier le sel de Na ;
- les acylglutamates mono- et disodique ;
- le sel de sodium de l'acide phosphatidique ;

2) Les lipides amphotères, ces lipides amphotères étant, de préférence, des phospholipides, en particulier la phosphatidyléthanolamine de soja pure ;

3) Les dérivés alkylsulfoniques, ces dérivés étant, de préférence, les composés de formule :

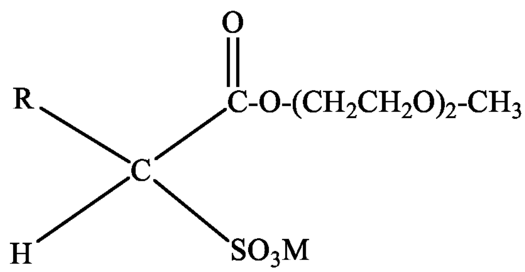

formule dans laquelle R représente les radicaux $C_{16}H_{37}$ et $C_{18}H_{37}$ pris en mélange ou séparément et M est un métal alcalin, de préférence le sodium.

On peut, de façon connue, incorporer dans la phase lipidique constituant la membrane lipidique des vésicules, au moins un additif qui a pour fonction principale de diminuer la perméabilité des vésicules, de prévenir leur floculation et leur fusion et d'augmenter le taux d'encapsulation.
Selon l'invention, on peut ajouter à la phase lipidique au moins un additif choisi, de préférence, dans le groupe formé par

- les stérols et notamment les phytostérols et le cholestérol,
- les alcools et diols à longue chaîne,
- les amines à longue chaîne et leurs dérivés ammonium quaternaire.

  • Les tensioactifs siliconés : comme tensioactifs siliconés pouvant être utilisés, on peut citer ceux décrits dans les documents US-A-5364633 et US-A-5411744. Ces documents décrivent l'utilisation de tensioactifs siliconés pour préparer des vésicules.

De préférence, le tensioactif siliconé utilisé selon la présente invention est un composé de formule (II) :

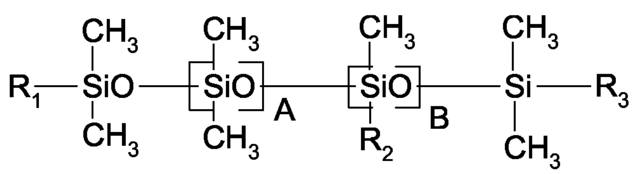

(II)

dans laquelle

$R_1$, $R_2$, $R_3$, indépendamment les uns des autres, représentent un radical alkyle en $C_1$-$C_6$ ou un radical $-(CH_2)_x-(OCH_2CH_2)_y-(OCH_2CH_2CH_2)_z-OR_4$, au moins un radical $R_1$, $R_2$ ou $R_3$ n' étan t pas un radical alkyle ; $R_4$ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (II), le radical alkyle est un radical méthyle, x est un nombre entier allant de 2 à 6 et y est un nombre entier allant de 4 à 30.
On peut citer, à titre d'exemple de tensioactifs siliconés de formule (II), les composés de formule (III) :

$$(CH_3)_3SiO - [(CH_3)_2SiO]_A - (CH_3SiO)_B - Si(CH_3)_3$$

$$(CH_2)_2-(OCH_2CH_2)_y-OH \qquad \text{(III)}$$

dans laquelle A est un nombre entier allant de 20 à 105, B est un nombre entier allant de 2 à 10 et y est un nombre entier allant de 10 à 20.

On peut également citer à titre d'exemple de tensioactifs siliconés de formule (II), les composés de formule (IV) :

$$HO - (OCH_2CH_2)_y - (CH_2)_3 - [(CH_3)_2SiO]_{A'} - (CH_2)_3 - (OCH_2CH_2)_y - OH \quad (IV)$$

dans laquelle A' et y sont des nombres entiers allant de 10 à 20.

On peut utiliser comme composés de l'invention ceux vendus par la société Dow Corning sous les dénominations DC 5329, DC 7439-146, DC 2-5695 et Q4-3667. Les composés DC 5329, DC 7439-146, DC 2-5695 sont des composés de formule (III) où respectivement A est 22, B est 2 et y est 12 ; A est 103, B est 10 et y est 12 ; A est 27, B est 3 et y est 12.

Le composé Q4-3667 est un composé de formule (IV) où A est 15 et y est 13.

→ Sont par exemple capables de former des phases lamellaires les mélanges binaires ou ternaires d'amphiphiles suivants (nom CTFA) :

- A/ cholestérol / lipoaminoacide caséique notamment dans un rapport pondéral 45/45/10 (où A est un cétyléther de triglycéryle commercialisé par la société Chimex sous le nom Chimexane NL) ;

- B/ cholestérol / dicétylphosphate notamment dans un rapport pondéral 60/35/5 (où B est un mélange de mono, di et tricétyléther de triglycéryle commercialisé par la société Chimex sous le nom Chimexane NT) ;

- Span 40 (de chez Ici ou Sorbitan palmitate)/cholestérol / acylglutamate de sodium (vendu par la Société Aj inomoto sous le nom HS 11) notamment dans un rapport pondéral 47,5/47,5/5 ;

- Stéarate de PEG 8 / cholestérol / acylglutamate de sodium avec notamment un rapport pondéral 47,5/47,5/5 (où le stéarate de PEG 8 est le polyéthylène glycol à 8 motifs d'oxyde d'éthylène commercialisé par la société Unichema sous le nom Stéarate PEG 400) ;

- Stéarate de PEG 8 / cholestérol / phytanetriol / acylglutamate de sodium avec notamment un rapport pondéral 47,5/20/27,5/5 ;

- Lécithine hydrogénée / phytostérol polyoxyéthyléné à 5 motifs d'oxyde d'éthylène notamment dans un rapport pondéral 60/40 ;

- distéarate de méthylglucose polyoxyéthyléné à 20 motifs d'oxyde d'éthylène / cholestérol / acylglutamate de sodium notamment dans un rapport pondéral 45/45/10 (le distéarate étant par exemple vendu sous le nom Glucam E 20 distéarate par Amerchol) ;

- A / cholestérol / dicétylphosphate avec notamment un rapport pondéral 47,5/47,5/5 ;

- distéarate de diglycéryle (par exemple celui vendu par Nihon sous le nom Emalex DS G2) /cholestérol / acylglutamate de sodium dans un rapport pondéral 45/45/10 ;

- mono et distéarate de saccharose (par exemple celui vendu par Grillo sous le nom Grilloten PSE 141 G) / cholestérol / acylglutamate de sodium, notamment dans un rapport pondéral 45/45/10 ;

- tristéarate de tétraglycéryle (par exemple celui vendu par Nikkol sous le nom Tetraglyn 3S) / cholestérol / acylglutamate de sodium, notamment dans un rapport pondéral de 45/45/10.

On peut citer comme exemples de vésicules de la deuxième catégorie (délivrant l'actif dans les couches superficielles de la peau) les vésicules obtenues à partir des lipides suivants :

- Lécithine de tournesol ;
- Natipide II (lécithine de soja / éthanol / eau dans un rapport pondéral 60/20/20 commercialisé par Nattermann) ;

- C (lécithine de soja / cholestérol / propylène glycol dans un rapport pondéral 40/30/30 commercialisé par Nattermann sous le nom NAT 50 PG) ;

- D / dimyristylphosphate notamment dans un rapport pondéral 95/5 (où D est un éther de lauryl polyglycéryl-6-cétéarylglycol commercialisé par Chimex sous le nom Chimexane NS).

→ sont capables également de former phases lamellaires lyotropes les polymères amphiphiles tels que les copolymères blocs amphiphiles à blocs asymétriques; par exemples ceux décrits dans les articles suivant : les diblocs copolymères amphiphiles :

- comme par exemple l'alcool éthoxylate dibloc copolymère $C_nE_n$ (C=méthylène, E= oxyethylène, l'indice n décrit le nombre d'unités répétées) décrits dans l'article: Cubic gels and lamellar crystals in concentrated solution of an amphiphilic diblock copolymer. I. W Hamley et Al. - A : Physicochemical and engineering Aspects 145 (1998), -185-190.

Les di ou multi-blocs amphiphiles, comme par exemple ceux décrits dans :

- Asymmetric amphiphilic block copolymers en solution: a morphological wonderland de Neil S. Cameron, Muriel K. Corbierre and A. Eisenberg. Canadian Journal of Chemistry, 1999, p.2-39.
- Block copolymer assembly to control fluid rheology de SR. Bhatia, A Mourchid, M. Joanicot. CurrentOpinion in Colloid & Interface Science, 6 (2001) 471-478.
- Thermodynamic size control of block copolymer vesicules in solution, de L. Luo and A. Eisenberg. Langmuir, 2001, 17, 6804-6811.
- Structural polymorhism of amphiphilic copolymers : six lyotropic liquid crystalline and two solution phases in a poly (oxybutylene)-b-poly(oxyethylene)-water-xylene- system. Langmuir, 1997, 13, 23-34.

Lorsque l'un au moins des agents structurants est une phase lamellaire, celle-ci peut être, en particulier, soit à base de tensioactif anionique, tel que le stéarate de triéthalonamine, soit à base de tensioactif non-ionique tel que le Peg-30 glycéryl stéarate (HLB=16,4 ;Mw=1678).

Les polymères associatifs peuvent être choisis parmi les polyuréthanes associatifs, les polymères acryliques associatifs, les polysaccharides associatifs.

Les polyuréthannes associatifs sont des copolymères séquencés non ioniques comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

En particulier, ces polymères comportent au moins deux chaînes lipophiles hydrocarbonées, ayant de $C_6$ à $C_{30}$ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polymères peuvent être séquencés sous forme de tribloc ou multibloc. Les séquences hydrophobes peuvent donc être à chaque extrémité de la chaîne (par exemple: copolymère tribloc à séquence centrale hydrophile) ou réparties à la fois aux extrémités et dans la chaîne (copolymère multiséquencé par exemple). Les polymères peuvent être également en greffons ou en étoile.

De préférence, les polymères sont des copolymères triblocs dont la séquence hydrophile est une chaîne polyoxyéthylénée comportant de 50 à 1 000 groupements oxyéthylénés. En général les polyuréthannes associatifs comportent une liaison uréthane entre les séquences hydrophiles, d'où l'origine du nom.

Par extension figurent aussi parmi les polyuréthannes associatifs, des polymères dont les séquences hydrophiles sont liées par d'autres liaisons chimiques que la liaison uréthane aux séquences lipophiles.

A titre d'exemple, des polymères associatifs utilisables dans l'invention, on peut citer le polymère $C_{16}$-$OE_{120}$-$C_{16}$ vendu par la société HULS (sous le nom Sérad FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 ou 204. Ces polyuréthannes associatifs sont vendus sous forme pure.

Le produit DW 1206B de chez ROHM & HAAS à chaîne alkyle en $C_{20}$ et à liaison uréthane, vendu à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Sérad FX1010, le Sérad FX1035 et le Serad 1070 vendus par la société HULS, le Rhéolate 255, le Rhéolate 278 et le Rhéolate 244 vendus par la société RHEOX.

On peut aussi utiliser le produit DW 1206F et le DW 1206J, ainsi que l'Acrysol RM 184 ou l'Acrysol 44 de la société ROHM & HAAS, ou bien encore le Borchigel LW 44 de la société BORCHERS.

Les polymères utilisables dans l'invention sont en particulier ceux décrits dans l'article de G. Fonnum, J. Bakke et Fk. Hansen - Colloid Polym. Sci 271, 380.389 (1993).

L'agent structurant de phase aqueuse peut être présent dans la composition en une teneur allant de 0,1 à 15% en poids, par rapport au poids total de la composition, (notamment de 0,1 à 8%) de préférence allant de 0,5 à 12% en poids, (notamment de 0,5% à 5%, ou de 0,8% à 3% en poids) et mieux allant de 1% à 8% en poids.

La composition selon l'invention peut comprendre un mélange d'un agent structurant de phase grasse et un agent structurant de phase aqueuse tels que définis dans les modes de réalisation décrits précédemment.

La composition selon l'invention forme un milieu physiologiquement acceptable.

Dans la présente demande, on entend par "milieu physiologiquement acceptable", un milieu non toxique compatible avec les fibres kératiniques d'êtres humains, notamment les cils ou les sourcils, comme un milieu cosmétique, le milieu cosmétique pouvant être un milieu cosmétique hydrophile ou lipophile.

La composition peut comprendre de l'eau ou un mélange d'eau et de solvant(s) organique (s) hydrophile (s), c'est-à-dire un ou des solvant (s) organique(s) miscibles à l'eau, comme les alcools et notamment des monoalcools ayant de 2 à 5 atomes de carbone comme l'éthanol, l'isopropanol ou le n-propanol, les polyols ayant de 2 à 8 atomes de carbone comme la glycérine, la diglycérine, le propylène glycol, l'éthylène glycol, le 1,3-butylène glycol, le sorbitol, le penthylène glycol, les cétones en $C_3$-$C_4$, les aldéhydes en $C_2$-$C_4$.

L'eau ou le mélange d'eau et de solvant(s) organique(s) hydrophile(s) peut être présent dans la composition selon l'invention en une teneur allant de 0,1 % à 90 % en poids, par rapport au poids total de la composition, et de préférence de 0,1 % à 60 % en poids.

Selon un autre mode de réalisation, la phase grasse telle que définie précédemment peut former une phase continue de la composition. En particulier, la composition selon l'invention peut être anhydre.

La composition selon l'invention peut être une composition de maquillage, une base de maquillage (dite « base-coat »), une composition dite « top-coat » à appliquer sur un maquillage, ou une composition de traitement cosmétique, de soin des fibres kératiniques.

La composition selon l'invention s'applique plus particulièrement aux cils. De ce fait, la composition de l'invention peut être une composition de revêtements des cils, notamment une composition de maquillage des cils, encore appelé mascara, une composition à appliquer sur un maquillage des cils, dite encore top-coat, ou bien encore une composition de traitement des cils, notamment des cils d'êtres humains ou des faux cils. Plus spécialement, la composition est un mascara.

L'invention a trait également a un procédé cosmétique de traitement ou de maquillage des fibres kératiniques, comprenant l'application sur lesdites matières kératiniques de la composition, telle qu'elle est décrite plus haut.

L'invention concerne aussi un procédé de revêtement des cils comprenant l'application sur les cils de la composition décrite ci-dessus.

L'invention est également relative à l'utilisation de la composition, telle que décrite plus haut, pour le maquillage des fibres kératiniques ainsi qu'à l'utilisation de cette composition pour obtenir une application facile et homogène, un maquillage présentant un excellent effet volumateur et pour obtenir un maquillage rapide des fibres kératiniques.

La composition selon l'invention peut comprendre en outre une cire additionnelle, différente de la cire collante décrite précédemment.

La cire additionnelle peut être choisie parmi par exemple parmi la cire d'abeille, les cires de paraffine, l'huile de ricin hydrogénée, les cires de silicone.

Les cires (la cire collante et/ou les cires additionelles) présentes dans la composition peuvent être dispersées sous forme de particules dans un milieu aqueux. En particulier, la cire peut être présente sous forme d'émulsion cire-dans-eau.

La ou les cire (s) (la cire collante et/ou la ou les cire (s) additionelle (s)) présente (s) dans la composition peuvent aussi se présenter sous forme d'une microdispersion aqueuse de particules de cire. On entend par microdispersion aqueuse de cire, une dispersion aqueuse de particules de cire, dans laquelle la taille desdites particules de cire est inférieure ou égale à environ 1 μm.

Les microdispersions de cire sont des dispersions stables de particules colloïdales de cire, et sont notamment décrites dans « Microemulsions Theory and Practice », L.M.Prince Ed., Academic Press (1977), pages 21-32.

En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.

Les microdispersions de cire peuvent également être obtenues par agitation du mélange de cire, de tensioactif et d'eau à l'aide de moyens d'agitation tels que les ultrasons, l'homogénéisateur haute pression, les turbines.

Les particules de la microdispersion de cire ont de préférence des dimensions moyennes inférieures à 1 µm (notamment allant de 0, 02 µm à 0,99 µm), de préférence inférieures à 0,5 µm (notamment allant de 0,06 µm à 0,05 µm).

Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

La cire additionnelle peut être présente dans la composition selon l'invention en une teneur allant de 0,1 % à 50 % en poids, par rapport au poids total de la composition, de préférence de 0,5 % à 30 % en poids, et mieux de 1 % à 20 % en poids.

La composition selon l'invention peut comprendre au moins un composé gras pâteux à température ambiante. Par "corps gras pâteux" au sens de l'invention, on entend des corps gras ayant un point de fusion allant de 20 à 55 °C, de préférence 25 à 45°C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

De préférence, ces corps gras sont des composés hydrocarbonés, éventuellement de type polymérique ; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Le corps gras pâteux peut être présent dans la composition selon l'invention en une teneur allant de 0,01 à 60% en poids, par rapport au poids total de la composition, de préférence allant de 0,5 à 45 % en poids, et mieux allant de 2 % à 30 % en poids, dans la composition.

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :

- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de $C_1$-$C_6$ alkyl glucose polyoxyéthylénés, et leurs mélanges.
- parmi les tensioactifs anioniques : les acides gras en $C_{16}$-$C_{30}$ neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition selon l'invention peut comprendre au moins un polymère filmogène.

Le polymère filmogène peut être choisi dans le groupe formé par les polymères vinyliques, les polyuréthanes, les polyesters, les polyamides, les polyurées, et les polymères cellulosiques.

Le polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Dans la présente demande, on entend par "polymère filmogène", un polymère apte à former à lui seul ou en présence d'un agent auxiliaire de filmification, un film continu et adhérent sur un support, notamment sur les matières kératiniques comme les cils.

Le ou les polymères filmogènes qui peuvent être présents dans la composition de l'invention sont différents du « polymère semi-cristallin » tel qu'il a été défini plus haut.

De préférence, le ou les polymères filmogènes ne comprennent pas de motifs cristallisables. Dans le cas où ils en comprendraient, lesdits motifs cristallisables représenteraient moins de 30% en poids du poids total du polymère.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$ - éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth) acrylique (encore appelé les (méth) acrylates) , notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides: l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un

diol choisi parmi: l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, K+, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane diméthanol, acide isophtalique, acide sulfoisophtalique.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère filmogène peut être un polymère hydrosoluble et peut être présent dans une phase aqueuse de la composition; le polymère est donc solubilisé dans la phase aqueuse de la composition. Comme exemples de polymères filmogènes hydrosolubles, on peut citer :

- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle; les copolymères de vinylpyrrolidone et de caprolactame ; l'alcool polyvinylique ;
- les polymères d'origine naturelle, éventuellement modifiés, tels que:

  . les gommes arabiques, la gomme de guar, les dérivés du xanthane, la gomme de karaya ;
  . les alginates et les carraghénanes ;
  . les glycoaminoglycanes, l'acide hyaluronique et ses dérivés ;
  . la résine shellac, la gomme de sandaraque, les dammars, les élémis, les copals ;
  . l'acide désoxyribonucléïque ;
  . les muccopolysaccharides tels les chondroïtines sulfate,

  et leurs mélanges.

Selon une autre variante de réalisation de la composition selon l'invention, le polymère filmogène peut être un polymère solubilisé dans une phase grasse liquide comprenant des huiles ou solvants organiques tels que ceux décrits précédemment (on dit alors que le polymère filmogène est un polymère liposoluble). Par "phase grasse liquide", on entend, au sens de l'invention, une phase grasse liquide à température ambiante (25°C) et pression atmosphérique (760 mm de Hg, soit 105 Pa), composée d'un ou plusieurs corps gras liquides à température ambiante, appelés aussi huiles, généralement compatibles entre eux.

De préférence, la phase grasse liquide comprend une huile volatile, éventuellement en mélange avec une huile non volatile, les huiles pouvant être choisies parmi les huiles citées précédemment.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui ont pour but qui peuvent être soit du type

vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de la copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2232303 ; ils peuvent avoir un poids moléculaire moyen en poids allant de 2 000 à 500 000 et de préférence de 4 000 à 200 000.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Selon une autre variante de réalisation de la composition de l'invention, le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations « Neocryl XK-90® », « Neocryl A-1070® », « Neocryl A-1090® », « Neocryl BT-62® », « Neocryl A-1079® » et « Neocryl A-523® » par la société AVECIA-NEORESINS, « Dow Latex 432® » par la société DOW CHEMICAL, « Daitosol 5000 AD® » par la société DAITO KASEY KOGYO; ou encore les dispersions aqueuses de polyuréthane vendues sous les dénominations « Neorez R-981® » et « Neorez R-974® » par la société AVECIA-NEORESINS, les « Avalure UR-405® », « Avalure UR-410® », « Avalure UR-425® », « Avalure UR-450® », « Sancure 875® », « Sancure 861® », « Sancure 878® » et « Sancure 2060® » par la société GOODRICH, « Impranil 85® » par la société BAYER, « Aquamere H-1511® » par la société HYDROMER ; les sulfopolyesters vendus sous le nom de marque « Eastman AQ® » par la société EASTMAN CHEMICAL PRODUCTS, les dispersions vinyliques comme le « Mexomère PAM » et aussi les dispersions acryliques dans l'isododécane comme le « Mexomère PAP » par la société CHIMEX.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou

d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

La composition de l'invention peut comprendre, en outre, tout additif usuellement utilisé en cosmétique tels que les antioxydants, les charges, les conservateurs, les parfums, les neutralisants, les épaississants, les tensioactifs, les actifs cosmétisques ou dermatologiques, les agents plastifiants, les agents de coalescence, les vitamines, et leurs mélanges.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique.

L'invention va maintenant être décrite en référence aux exemples suivants donnés à titre illustratif et non limitatif.

Avant d'entrer plus en détail dans l'exposé de ces exemples, nous allons expliciter les protocoles de mesure des différentes grandeurs permettant de définir la présente invention, à savoir la teneur en extrait sec, la valeur de collant, la dureté et la mesure du profil d'écoulement.

<u>Protocole de mesure du collant</u>

Ce protocole est valable à la fois pour déterminer le collant d'une cire et le collant de l'association d'un composé particulier avec au moins une huile, ledit composé particulier pouvant être un polymère semi-cristallin, un agent rhéologique de phase grasse.

Le collant de l'agent structurant de la phase grasse est mesuré à 20 °C à l'aide du texturomètre vendu sous la dénomination TA-XT2i par la société RHEO, équipé d'un mobile en polymère acrylique en forme de cône formant un angle de 45°, en mesurant l'évolution de la force (force de compression ou force d'étirement) (F) en fonction du temps, pendant l'opération suivante :

Le mobile est déplacé à la vitesse de 0,5 mm /s puis pénètre dans l'agent structurant jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s. Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

Pour effectuer la mesure du collant de l'agent structurant, l'agent structurant est fondu à une température égale au point de fusion de l'agent structurant + 10 °C. L'agent structurant fondu est coulé dans un récipient de 25 mm de diamètre et de 20 mm de profondeur. L'agent structurant est recristallisé à température ambiante (25 °C) pendant 24 heures de telle sorte que la surface de l'agent structurant soit plane et lisse, puis l'agent structurant est conservé pendant au moins 1 heure à 20 °C avant d'effectuer la mesure du collant.

<u>Protocole de mesure de la dureté</u>

Ce protocole est valable à la fois pour déterminer la dureté d'une cire et la dureté de l'association d'un composé particulier avec au moins une huile, ledit composé particulier pouvant être un polymère semi-cristallin, un agent rhéologique de phase grasse.

La dureté de l'agent structurant de la phase grasse est mesuré à 20 °C à l'aide du texturomètre vendu sous la dénomination TA-XT2i par la société RHEO, équipé d'un mobile en inox en forme de cylindre d'un diamètre de 2mm, en mesurant l'évolution de la force (force de compression ou force d'étirement) (F) en fonction du temps, pendant l'opération suivante :

Le mobile est déplacé à la vitesse de 0,1 mm /s puis pénètre dans l'agent structurant jusqu'à une profondeur de pénétration de 0,3 mm. Lorsque le mobile a pénétré dans l'agent structurant à la profondeur de 0,3 mm, le mobile est maintenu fixe pendant 1 seconde (correspondant au temps de relaxation) puis est retiré à la vitesse de 0,1 mm/s. Pendant le temps de relaxation, la force (force de compression) décroît fortement jusqu'à devenir nulle puis, lors du

retrait du mobile, la force (force d'étirement) devient négative pour ensuite croître à nouveau vers la valeur 0. La dureté correspond à la force de compression maximale mesurée entre la surface du mobile et la cire au moment de leur mise en contact. La valeur de cette force est exprimée en MPa.

Pour effectuer la mesure de la dureté de l'agent structurant, l'agent structurant est fondu à une température égale au point de fusion de l'agent structurant + 20°C. L'agent structurant fondu est coulé dans un récipient de 30 mm de diamètre et de 20 mm de profondeur. L'agent structurant est recristallisé à température ambiante (25°C) pendant 24 heures de telle sorte que la surface de l'agent structurant soit plane et lisse, puis l'agent structurant est conservé pendant au moins 1 heure à 20°C avant d'effectuer la mesure de la dureté.

Protocole de mesure du profil d'écoulement

Les conditions de détermination du profil rhéologique mentionné ci-dessus sont les suivantes.

Les mesures ont été effectuées sur un rhéomètre à contrainte imposée, RS 75 de ThermoRhéo, équipé d'un bain thermostaté. La géométrie de cisaillement employée possède une symétrie plan / plan, de diamètre 2 cm, avec la surface des plans qui soit striée pour limiter les phénomènes de glissement à la paroi des plans.

Le gap (ou l'épaisseur d'échantillon) est fixé à 300 µm.

Les mesures sont effectuées à 25°C ± 0.5°C.

*Principe de la mesure*

L'analyse en régime d'écoulement à l'équilibre consiste à soumettre, à partir d'un instant donné, un échantillon à une contrainte $\tau$ instantanée, maintenue constante pendant un temps t (temps d'attente choisi de façon à ce que le régime permanent soit atteint, t = 30s). Simultanément, on suit l'évolution au cours du temps de la déformation de cisaillement correspondante $\gamma$ et on enregistre le gradient de cisaillement $\dot{\gamma}$ lorsque l'équilibre est atteint.

*Protocole.*

1ère étape: Mise en température de l'échantillon à 25°C pendant 2 min (sans aucun cisaillement)

2ème étape: Mesure en écoulement à l'équilibre - en mode contrainte imposée

Les conditions d'applications des contraintes sont les suivantes :

Contrainte initiale = 0.64 Pa
Contrainte finale = 2 000 Pa
Points mesurés = 40
Distribution des points = Logarithmique

On applique des contraintes croissantes à l'échantillon, les contraintes n'étant appliquées qu'une seule fois.

Attendre une valeur stable entre chaque contrainte.

Temps d'attente entre chaque contrainte = 30s.

*Analyse des résultats*

L'analyse des résultats se fait au travers de la représentation graphique de l'évolution de la viscosité, notée $\eta$, en fonction du gradient de cisaillement, noté $\dot{\gamma}$. Ce graphe montre une première zone plateau, appelée première région newtonienne, définie pour les faibles valeurs de $\dot{\gamma}$ ($\dot{\gamma} \leq 10^{-3} s^{-1}$) où la viscosité reste constante: cette zone est représentative de la viscosité du produit au repos et peut être rapprochée de la notion de consistance du mascara.

Pour les gradients de cisaillement plus élevés, $10^{-2} s^{-1} \leq \dot{\gamma} \leq 10^{3} s^{-1}$, la viscosité chute, le produit se met en mouvement pour s'écouler en se fluidifiant. Dans cette fenêtre de cisaillement, deux comportements peuvent apparaître :

- soit l'écoulement est homogène, au quel cas il y a équidistance entre les points du graphe et la courbe est telle que l'ensemble des rapports:

$$\frac{\Delta\dot{\gamma}}{\Delta\tau} \leq 7$$

- soit l'écoulement est inhomogène et dans ce cas il n'y a plus continuité ni équidistance entre les points du graphe et la courbe est telle que certains rapports:

$$\frac{\Delta\dot{\gamma}}{\Delta\tau} > 7$$

Protocole de mesure de la teneur en solides

Consiste en une mesure de l'extrait sec du jus de mascara réalisé sur une balance Mettler Toledo HG 53 (Halogen Moisture Analyzer).

Un échantillon de mascara (2-3g) est déposé sur une coupelle en aluminium et subit une température de 120°C pendant 60 minutes. La mesure de l'extrait sec correspond au suivi de la masse de l'échantillon en fonction du temps .La teneur finale en solides est donc le pourcentage de la masse finale (au bout de 60 min) par rapport à la masse initiale : ES = (masse finale /masse initiale) X 100, ES correspondant à la teneur en solides définie par un extrait sec en solides.

L'invention va maintenant être décrite en référence aux exemples suivants donnés à titre illustratif et non limitatif.

## BRÈVE DESCRIPTION DES DESSINS

La figure 1 représente le profil d'écoulement d'une composition de type mascara conforme à l'invention, ledit profil représentant la viscosité (η en Pa.s) en fonction du gradient de cisaillement $\dot{\gamma}$ (en $s^{-1}$).

La figure 2 représente le profil d'écoulement d'un mascara commercial Illusionist® par Estée Lauder, ledit profil représentant la viscosité (η en Pa.s) en fonction du gradient de cisaillement $\dot{\gamma}$ (en $s^{-1}$).

La figure 3 représente le profil d'écoulement d'une composition conforme à l'invention, ledit profil représentant la viscosité (η en Pa.s) en fonction du gradient de cisaillement $\gamma$ (en $s^{-1}$).

La figure 4 représente le profil d'écoulement d'un mascara commercial Long Optic@ de Dior, ledit profil représentant la viscosité (η en Pa.s) en fonction du gradient de cisaillement $\dot{\gamma}$ (en $s^{-1}$).

La figure 5 représente le profil d'écoulement d'un mascara commercial Effet Faux Cils@ d'Yves Saint-Laurent, ledit profil représentant la viscosité (η en Pa.s) en fonction du gradient de cisaillement $\dot{\gamma}$ (en $s^{-1}$).

EXEMPLES.

EXEMPLE 1.

Mascara émulsion cire dans eau avec écoulement homogène et continu

| Composition | |
|---|---|
| Acide stéarique | 5,82% |
| Cire de candellila | 6% |
| Hydroxystearoyl stéarate d'alcools gras en C20-C40 (Kester wax K82P) | 25% |
| Amino-2 methyl-2 propane di-ol-1,3 | 0,5% |
| Oxyde de fer noir | 5,45% |
| Silice | 3% |
| Hydroxyéthylcellulose | 0,91% |
| Gomme arabique | 3,45% |
| Tri-ethanolamine | 2,4% |
| Anti-mousse | qs |
| Conservateur | qs |
| Eau | qsp 100 |

Extrait sec mesuré = 53%

Pour cet exemple,

$$\frac{\Delta\dot{\gamma}}{\Delta\tau}$$

varie de $4.5.10^{-4}$ à 4.3 dans la gamme des cisaillements compris entre $1.10^{-4}$ $s^{-1}$ et $1.10^{3}s^{-1}$

Le mascara de cet exemple est capable de supporter des cisaillements élevés de l'ordre de $1000s^{-1}$.

La figure 1 représente le profil d'écoulement de ladite composition, représentant la viscosité de la composition en fonction du gradient de cisaillement.

Elle confirme que l'écoulement est continu et homogène et que cette composition est apte à subir une contrainte jusqu'à un gradient de cisaillement de $10^{-3}$ $s^{-1}$.

On obtient un maquillage chargeant les cils, lisse et homogène

EXEMPLE COMPARATIF 1.

La composition soumise au protocole de mesure de profil rhéologique précité est une composition commerciale Illusionist® d'Estée Lauder.

Extrait sec mesuré = 46.8%

Pour cette composition,

$$\frac{\Delta\dot{\gamma}}{\Delta\tau}$$

varie de $4,35.10^{-4}$ à 8.5 dans la gamme des cisaillements compris entre $1.10^{-2}s^{-1}$ et $1.10^{3}s^{-1}$. Dans la mesure où ce rapport dépasse 7, l'écoulement est donc discontinu et inhomogène.

- le mascara Illusionist n'est pas capable de supporter des cisaillements de 1 $000s^{-1}$ puisque $\dot{\gamma}_{max} = 400s^{-1}$.

La figure 2 confirme pour ce type de mascara un écoulement discontinu et non homogène.

Ce mascara confère un maquillage nature (peu épais) aux cils et forme un dépôt granuleux.

EXEMPLE 2.

Mascara émulsion cire dans eau avec tensioactif non ionique (correspondant à une phase lamellaire) exclusivement et présentant un écoulement homogène et continu

| Composition: | |
|---|---|
| Cire d'abeille | 4,63% |
| Huile de jojoba hydrogénée | 0,11% |
| Cire de carnauba | 3,41% |
| Cire de son de riz | 0,11% |
| Cire de paraffine | 14,64% |
| Cire de candellila | 0,32% |
| Oxyde de fer noir | 7,42% |
| Hydroxyéthyl cellulose | 0,94% |
| Gomme arabique | 3,59% |
| Monostéarate de glycéryl 300E (= Tagat S) | 2,54% |
| Mélange de poly dimethylsiloxane à groupements alpha-oméga hydroxyle et de cyclopentadimethylsiloxane (15/85) | 8,5% |
| Alcool stearylique 2 OE | 1,5% |

(suite)

| Composition: | |
|---|---|
| Alcool stearylique 20 OE | 1% |
| Anti-mousse | qs |
| Conservateur | qs |
| Eau | qsp |

Extrait sec mesuré = 45,24%

Pour cet exemple,

$$\frac{\Delta\dot{\gamma}}{\Delta\tau}$$

varie de $1,5.10^{-3}$ à 3,9 dans la gamme des cisaillements compris entre $1.10^{-2}$s-1 et $1.10^{3}$s-1. Ce mascara présente donc un écoulement continu et homogène.

Ce mascara de l'exemple 1 est capable de supporter des cisaillements élevés de l'ordre de 1000s-1, ce qui est confirmé par la figure 3.

Ce mascara confère de bonnes propriétés volumateur (maquillage épais des cils) et forme un dépôt lisse et homogène.

EXEMPLE COMPARATIF 2: Long'Optic de Dior

Extrait Sec = 42,5% mesuré

$$\frac{\Delta\dot{\gamma}}{\Delta\tau}$$

varie de $1,2.10^{-3}$ à 30, 8 dans la gamme des cisaillements compris entre $1.10^{-2}$ $s^{-1}$ et $1.10^{3}$ $s^{-1}$.

Ce mascara ne rentre pas dans le cadre de notre invention car l'écoulement est inhomogène au-delà de $10^{-2}$ $s^{-1}$, ce qui est confirmé par la figure 4.

Ce mascara confère un maquillage naturel aux cils (maquillage peu épais) et forme un dépôt non lisse.

EXEMPLE COMPARATIF 3: Effet Faux cils d'Yves Saint Laurent

Extrait Sec = 52,7% mesuré

$$\frac{\Delta\dot{\gamma}}{\Delta\tau}$$

varie de $1,8.10^{-3}s^{-1}$ à 7,5 dans la gamme des cisaillements compris entre $1.10^{-2}$ $s^{-1}$ et $1.10^{3}$ $s^{-1}$.

Le mascara EFC ne rentre pas dans le cadre de notre invention, car l'écoulement est inhomogène au-delà de 10 s-1, ce qui est confirmé par la figure 5.

Ce mascara confère un maquillage chargeant des cils mais forme un dépôt inhomogène.

**Revendications**

**1.** Composition de maquillage de fibres kératiniques, présentant:

- une teneur en solides définie par un extrait sec en solides supérieur à 45 % du poids total de la composition; et

- un profil rhéologique tel que, pour une gamme de gradient de cisaillement allant $10^{-2}$ à $10^{3}$ $s^{-1}$, les rapports de la variation du gradient de cisaillement sur la variation de la contrainte de cisaillement appliquée (noté

$$\frac{\Delta\dot{\gamma}}{\Delta\tau})$$

sont au plus égaux à 7 ; ladite composition étant apte à subir des contraintes de cisaillement sans fracturation dans toute la largeur de ladite gamme de gradient de cisaillement.

**2.** Composition selon la revendication 1, dans laquelle lesdits rapports sont inférieurs à 5, de préférence inférieurs à 4,5, mieux encore inférieurs à 1,5.

**3.** Composition selon la revendication 1 ou 2, dans laquelle l'extrait sec en solides est supérieur à 46 %, de préférence supérieur à 47 %, encore plus préférentiellement supérieur à 48 % ou mieux encore supérieur à 50 % du poids total de la composition.

**4.** Composition selon l'une quelconque des revendications 1 à 3, comprenant au moins une phase grasse comprenant au moins un agent structurant de phase grasse.

**5.** Composition selon la revendication 4, dans laquelle l'agent structurant est choisi parmi les cires collantes, les associations d'un composé particulier avec au moins une huile et leurs mélanges.

**6.** Composition selon la revendication 5, dans laquelle les cires collantes présentes les caractéristiques suivantes :

- un collant ≥ 0,7 N.s, notamment allant de 0,7 à 30 N.s ; de préférence ≥ 1 N.s, notamment allant de 1 à 20 N.s ; mieux encore ≥ 2 N.s, notamment allant de 2 à 10 N.s ;
- une dureté ≤ 3,5 MPa, de préférence allant de 0,01 à 3,5 MPa, encore plus préférentiellement de 0,05 à 3 MPa et mieux encore de 0,1 à 2,5 MPa.

**7.** Composition selon la revendication 5, dans laquelle ledit agent structurant consiste en l'association d'un composé particulier avec au moins une huile.

**8.** Composition selon la revendication 7, dans laquelle le composé particulier est choisi parmi les polymères semi-cristallins, les agents rhéologiques de phase grasse; et leurs mélanges.

**9.** Composition selon la revendication 7 ou la revendication 8, dans laquelle l'huile est choisie parmi les huiles hydrocarbonées, siliconées, et/ou fluorées volatiles et non-volatiles, et leurs mélanges.

**10.** Composition selon l'une quelconque des revendications 7 à 9, dans laquelle l'huile présente une masse moléculaire supérieure ou égale à 250 g/mol, notamment de 250 à 10000 g/mol, de préférence supérieure ou égale à 300g/mol, notamment de 300 à 8000 g/mol et mieux, supérieure ou égale à 400 g/mol, notamment de 400 à 5000 g/mol.

**11.** Composition selon l'une quelconque des revendications 7 à 10, dans laquelle l'association d'un composé particulier avec au moins une huile présente:

- un collant ≥ 0,1 N.s, notamment allant de 0,1 à 30 N.s ; de préférence ≥ 0,5 N.s, notamment allant de 0,5 à 20 N.s ; ou mieux encore ≥ 1 N.s, notamment allant de 1 à 10 N.s ;
- une dureté ≤ 30 MPa, notamment allant de 0,01 à 30 MPa, de préférence de 0, 05 à 25 MPa, ou encore plus préférentiellement de 0,1 à 20 MPa.

**12.** Composition selon la revendication 8, dans laquelle l'association polymère semi-cristallin avec au moins une huile présente un collant de 1 à 5 N.s et une valeur de dureté de 0,1 à 20 MPa.

**13.** Composition selon l'une quelconque des revendications 4 à 12, qui comprend de 0,1 à 60 %, de préférence de 5 à 55 % en poids, de préférence encore de 10 à 40 % en poids d'agent structurant.

**14.** Composition selon l'une quelconque des revendications 1 à 3, comprenant au moins une phase aqueuse comprenant au moins un agent structurant de phase aqueuse, ce dernier pouvant être choisi parmi les tensioactifs

non ioniques et anioniques conduisant à la formation de phases lamellaires, les polymères amphiphiles conduisant à la formation de phases lamellaires, les polymères associatifs.

**15.** Composition selon l'une quelconque des revendications 1 à 14, comprenant de l'eau ou un mélange d'eau et de solvant(s) organique (s) hydrophile (s).

**16.** Composition selon la revendication 15, dans laquelle le (s) solvant (s) organique (s) hydrophile (s) est (sont) choisi (s) parmi les monoalcools ayant de 2 à 5 atomes de carbone, les polyols ayant de 2 à 8 atomes de carbone, les cétones en $C_3$-$C_4$ et les aldéhydes en $C_2$-$C_4$.

**17.** Composition selon l'une quelconque des revendications 15 et 16, dans laquelle l'eau ou le mélange d'eau et de solvant (s) organique (s) hydrophile(s) est présent en une teneur allant de 0,1% à 90% en poids, par rapport au poids total de la composition, et de préférence de 0,1% à 60% en poids.

**18.** Composition selon l'une quelconque des revendications précédentes, **caractérisée en ce qu'**elle comprend un polymère filmogène.

**19.** Composition selon la revendication 18, dans laquelle le polymère filmogène est choisi dans le groupe formé par les polymères vinyliques, les polyuréthanes, les polyesters, les polyamides, les polyurées, et les polymères cellulosiques.

**20.** Composition selon la revendication 18 ou 19, dans laquelle le polymère filmogène est présent en une teneur en matières sèches de polymère allant de 0,1% à 60% en poids par rapport au poids total de la composition, de préférence de 0,5% à 40% en poids, et mieux de 1% à 30% en poids.

**21.** Composition selon l'une quelconque des revendications précédentes, **caractérisée en ce qu'**elle comprend une matière colorante.

**22.** Composition selon la revendication 21, dans laquelle la matière colorante est choisie parmi les pigments, les nacres, les colorants liposolubles, et les colorants hydrosolubles.

**23.** Composition selon la revendication 21 ou la revendication 22, dans laquelle la matière colorante est présente en une teneur allant de 0,01% à 30% en poids, par rapport au poids total de la composition.

**24.** Composition selon l'une quelconque des revendications précédentes, **caractérisée en ce qu'**elle comprend un additif cosmétique choisi parmi les antioxydants, les charges, les conservateurs, les parfums, les neutralisants, les épaississants, les tensioactifs, les actifs cosmétiques ou dermatologiques, les agents plastifiants, les agents de coalescence et leurs mélanges.

**25.** Composition selon l'une quelconque des revendications précédentes, qui est une composition de maquillage, une base de maquillage, une composition dite « top-coat » à appliquer sur un maquillage, ou une composition de traitement, de soin, des matières, fibres, kératinique.

**26.** Composition selon l'une quelconque des revendications précédentes qui est une composition de revêtement des cils, notamment une composition de maquillage des cils, encore appelé mascara, une composition à appliquer sur un maquillage des cils, dite encore top-coat, ou bien encore une composition de traitement des cils, notamment des cils d'êtres humains ou des faux cils.

**27.** Composition selon la revendication 26, qui est un mascara.

**28.** Procédé cosmétique de traitement ou de maquillage des fibres kératiniques comprenant l'application sur lesdites fibres kératiniques d'une composition selon l'une quelconque des revendications 1 à 27.

**29.** Procédé de revêtement des cils comprenant l'application sur les cils d'une composition selon l'une quelconque des revendications 1 à 27.

**30.** Utilisation d'une composition selon l'une quelconque des revendications 1 à 27 pour le maquillage des fibres kératiniques.

**31.** Utilisation d'une composition selon l'une quelconque des revendications 1 à 27, pour obtenir une application facile et homogène et un maquillage présentant un excellent effet volumateur et pour obtenir un maquillage rapide des fibres kératiniques.

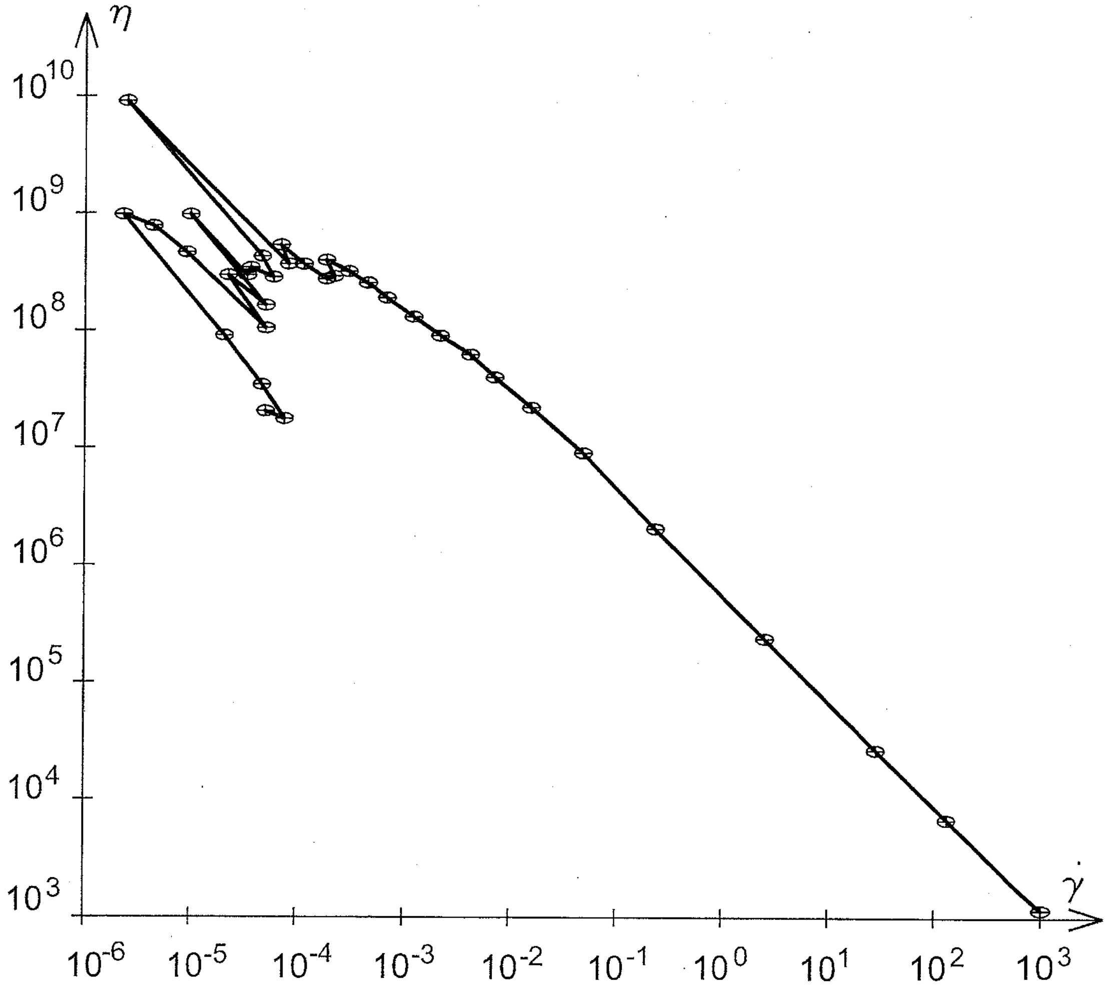
η
$10^{10}$
$10^9$
$10^8$
$10^7$
$10^6$
$10^5$
$10^4$
$10^3$
$\dot{\gamma}$
$10^{-6}$
$10^{-5}$
$10^{-4}$
$10^{-3}$
$10^{-2}$
$10^{-1}$
$10^0$
$10^1$
$10^2$
$10^3$

FIG. 1

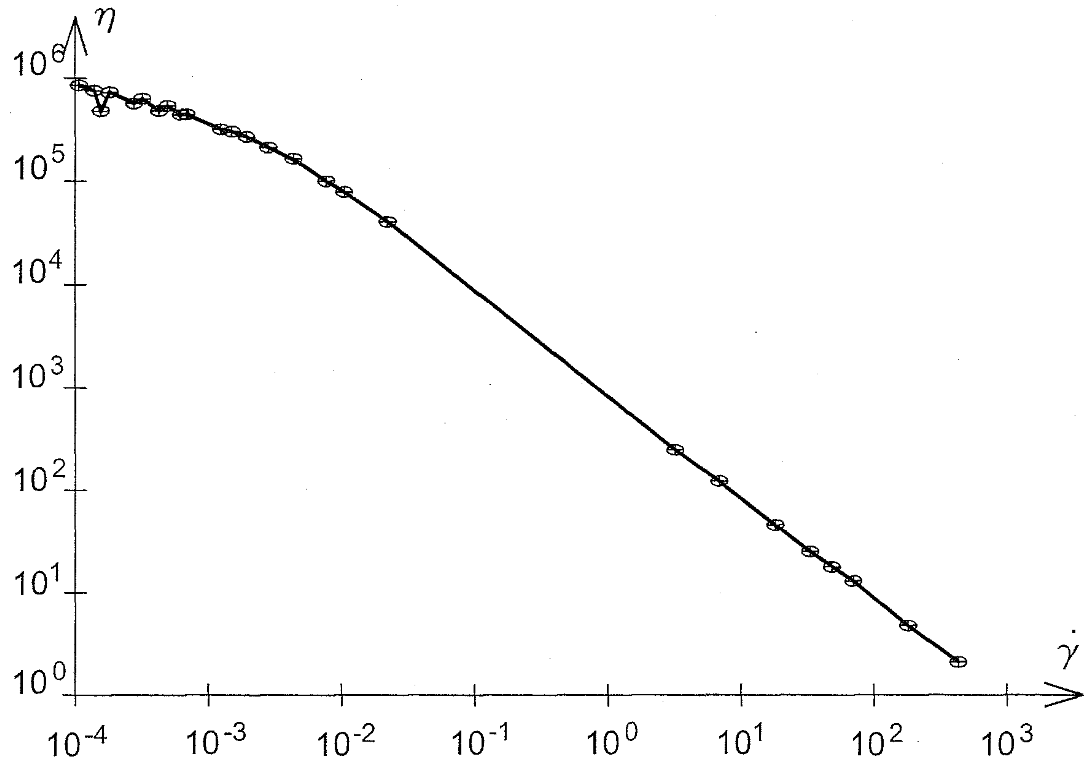
η
$10^6$
$10^5$
$10^4$
$10^3$
$10^2$
$10^1$
$10^0$
$\dot{\gamma}$
$10^{-4}$
$10^{-3}$
$10^{-2}$
$10^{-1}$
$10^0$
$10^1$
$10^2$
$10^3$

FIG. 2

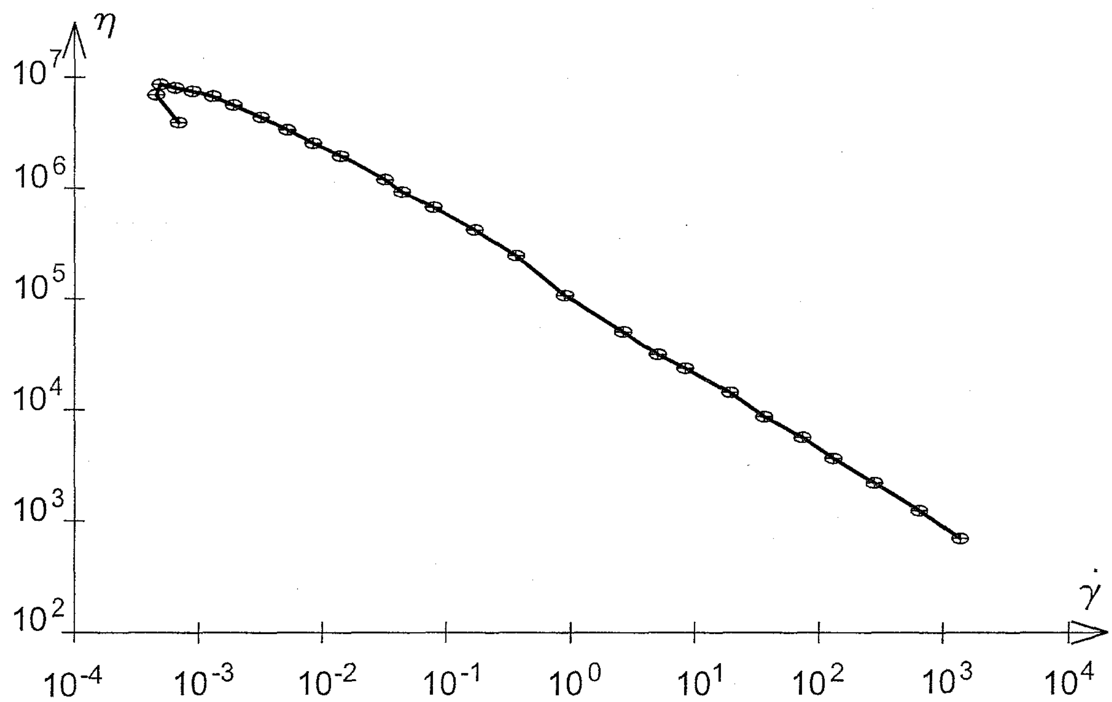
η
$10^7$
$10^6$
$10^5$
$10^4$
$10^3$
$10^2$
$\dot{\gamma}$
$10^{-4}$
$10^{-3}$
$10^{-2}$
$10^{-1}$
$10^0$
$10^1$
$10^2$
$10^3$
$10^4$

FIG. 3

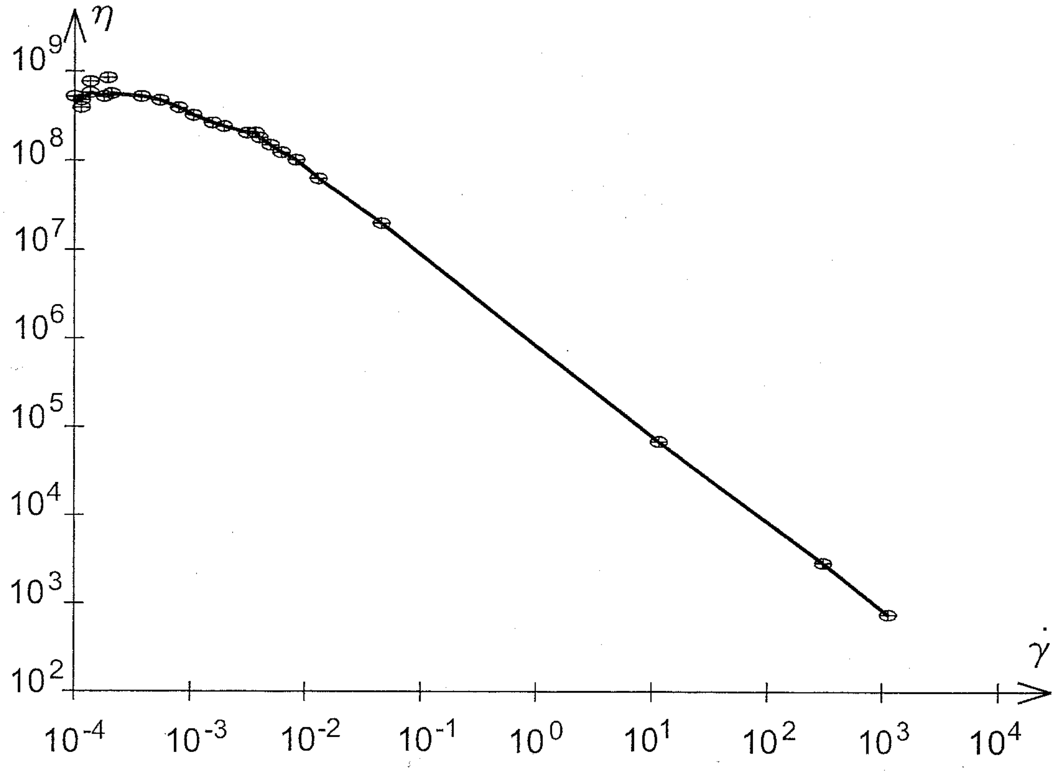
η
$10^9$
$10^8$
$10^7$
$10^6$
$10^5$
$10^4$
$10^3$
$10^2$
$\dot{\gamma}$
$10^{-4}$ $10^{-3}$ $10^{-2}$ $10^{-1}$ $10^0$ $10^1$ $10^2$ $10^3$ $10^4$

FIG. 4

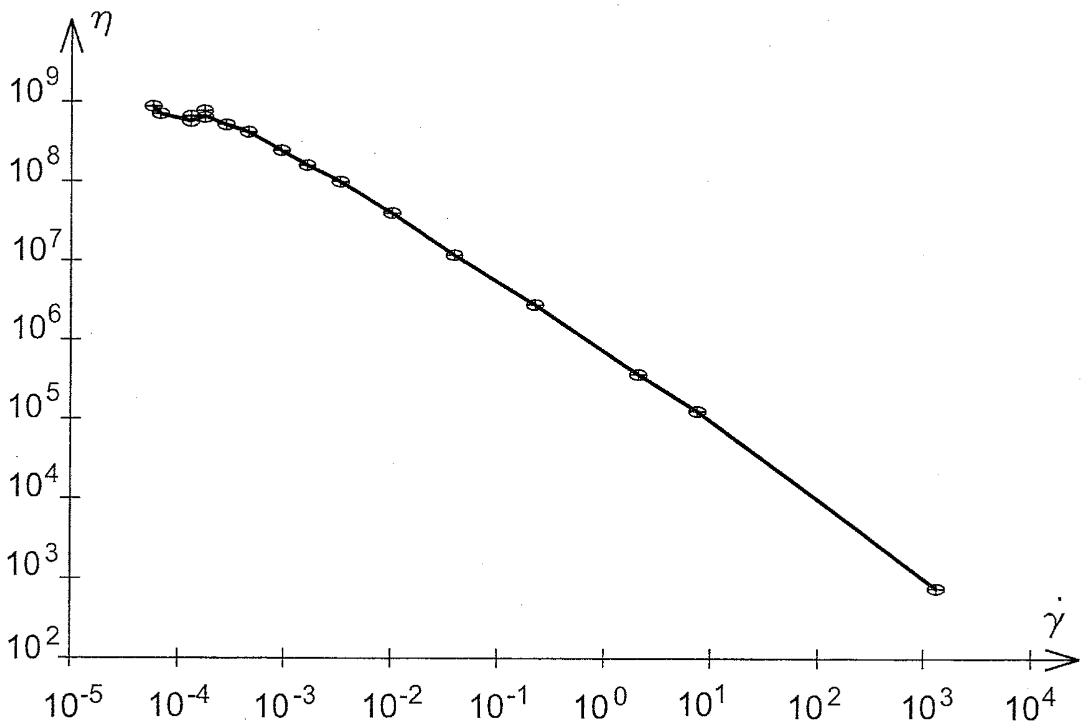
η
$10^9$
$10^8$
$10^7$
$10^6$
$10^5$
$10^4$
$10^3$
$10^2$
$\dot{\gamma}$
$10^{-5}$ $10^{-4}$ $10^{-3}$ $10^{-2}$ $10^{-1}$ $10^0$ $10^1$ $10^2$ $10^3$ $10^4$

FIG. 5